# EUROPEAN PATENT APPLICATION

(11) **EP 1 695 971 A1**
(43) Date of publication of application: **30.08.2006**
(21) Application number: 04380289.1
(22) Date of filing: 30.12.2004
(51) Int. Cl.: C07D 295/14, C07D 307/52, C07D 231/12, A61K 31/496, A61P 3/04

(54) **Substituted phenyl-piperazine compounds, their preparation and use in medicaments**

(71) Applicant: LABORATORIOS DEL DR. ESTEVE, S.A., 08041 Barcelona (ES)
(72) Inventor: Aschenbrenner, Andrea, D-81241 München (DE); Kraus, Jürgen, D-82319 Starnberg (DE); Tasler, Stefan, D-82205 Gilching (DE); Wuzik, Andreas, 86836 Untermeitingen (DE); Quintana-Ruiz, Jordi Ramon, E-08391 Tiana (Barcelona) (ES)
(74) Representative: Carvajal y Urquijo, Isabel

(57) **Abstract**

The present invention relates to substituted phenyl-piperazine compounds of general formula I, 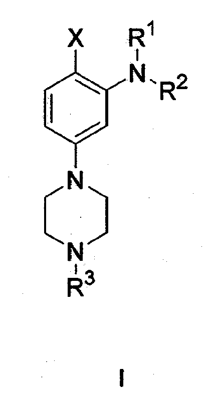 a process for their preparation, medicaments comprising said substituted phenyl-piperazine compounds as well as the use of said substituted phenyl-piperazine compounds for the preparation of medicaments, which are particularly suitable for the prophylaxis and/or treatment of disorders or diseases that are at least partially mediated via 5-HT₆ receptors.

## Description

The present invention relates to substituted phenyl-piperazine compounds of general formula I, 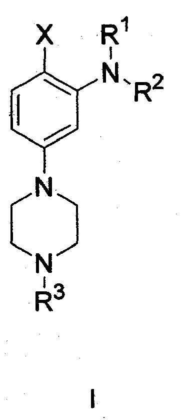 a process for their preparation, medicaments comprising said substituted phenyl-piperazine compounds as well as the use of said substituted phenyl-piperazine compounds for the preparation of medicaments, which are particularly suitable for the prophylaxis and/or treatment of disorders or diseases that are at least partially mediated via 5-HT₆ receptors.

The superfamily of serotonin receptors (5-HT) includes 7 classes (5-HT₁-5-HT₇) encompassing 14 human subclasses [D. Hoyer, et al., Neuropharmacology, 1997, 36, 419]. The 5-HT₆ receptor is the latest serotonin receptor identified by molecular cloning both in rats [F.J. Monsma et al., Mol. Pharmacol., 1993, 43, 320; M. Ruat et al., Biochem. Biophys. Res. Commun., 1993,193, 268] and in humans [R. Kohen, et al., J. Neurochem., 1996, 66, 47].

Compounds with 5-HT₆ receptor affinity are useful for the treatment of various disorders of the Central Nervous System and of the gastrointestinal tract, such as irritable intestine syndrome. Compounds with 5-HT₆ receptor affinity are also useful in the treatment of anxiety, depression and cognitive memory disorders [M. Yoshioka et al., Ann. NY Acad. Sci., 1998, 861, 244; A. Bourson et al., Br. J. Pharmacol. , 1998, 125, 1562; D.C. Rogers et al., Br. J. Pharmacol. Suppl., 1999, 127, 22P; A. Bourson et al., J. Pharmacol. Exp. Ther., 1995, 274, 173; A.J. Sleight, et al., Behav. Brain Res. , 1996, 73, 245; T.A. Branchek et al., Annu. Rev. Pharmacol. Toxicol. , 2000, 40, 319; C. Routledge et al., Br. J. Pharmacol. , 2000, 130, 1606]. It has been shown that typical and atypical antipsychotic drugs for treating schizophrenia have a high affinity for 5-HT₆ receptors [B.L. Roth et al., J. Pharmacol. Exp. Ther. , 1994, 268, 1403; C.E. Glatt et al., Mol. Med., 1995, 1, 398; F.J. Mosma,et al., Mol. Pharmacol. , 1993, 43, 320; T. Shinkai et al., Am. J. Med. Genet. , 1999, 88, 120]. Compounds with 5-HT₆ receptor affinity are useful for treating infant hyperkinesia (ADHD, attention deficit / hyperactivity disorder) [W.D. Hirst et al., Br. J. Pharmacol. , 2000, 130, 1597; C. Gérard et al., Brain Research , 1997, 746, 207; M.R. Pranzatelli, Drugs of Today , 1997, 33, 379].

Recently, it has been shown that the 5-HT₆ receptor also plays a role in food ingestion [Neuropharmacology, 41, 2001, 210-219].

Food ingestion disorders, particularly obesity, are a serious, fast growing threat to the health of humans of all age groups, since they increase the risk of developing other serious, even life-threatening diseases such as diabetes or coronary diseases as well.

Therefore an object of the present invention was to provide compounds that are particularly suitable as active ingredients in medicaments, especially in medicaments for the prophylaxis and/or treatment of disorders or diseases related to 5-HT₆ receptors such as food intake related disorders.

Surprisingly, it has been found that the substituted phenyl-piperazine compounds of general formula I given below show good to excellent affinity for 5-HT₆-receptors. These compounds are therefore particularly suitable as pharmacologically active agents in a medicament for the prophylaxis and/or treatment of disorders or diseases related to 5-HT₆-receptors such as food intake related disorders like obesity.

Thus, in one of its aspects the present invention relates to substituted phenyl-piperazine compounds of general formula I, 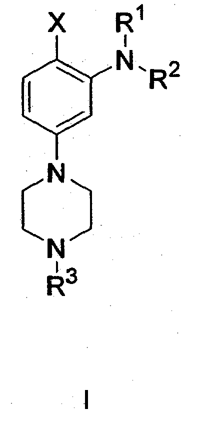 wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents a hydrogen atom;
a saturated or unsaturated 3- to 9-membered cycloaliphatic radical, which may be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl,-C(=O)-OH, -C(=O)-C₁₋₅-alkyl, -C(=O)-O-C₁₋₅-alkyl, -O-C(=O)-C₁₋₅-alkyl, F, Cl, Br, I,-CN, -CF₃, -OCF₃, -SCF₃, -OH, -SH, -NH₂, -NH(C₁₋₅-alkyl), -N(C₁₋₅-alkyl)₂, -NO₂,-CHO, -CF₂H, -CFH₂, -C(=O)-NH₂, -C(=O)-NH(C₁₋₅-alkyl), -C(=O)-N(C₁₋₅-alkyl)₂,-S(=O)₂-C₁₋₅-alkyl, -S(=O)₂-phenyl, phenyl, phenoxy and benzyl and which may contain1, 2 or 3 heteroatom(s) independently selected from the group consisting of nitrogen, oxygen and sulfur as ring member(s) and which is bonded via a linear or branched C₁₋₆ alkylene, C₂₋₆ alkenylene or C₂₋₆ alkinylene group which maybe unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅₋alkyl, -S-C₁₋₅-alkyl, -NH(C₁₋₅-alkyl) and -N(C₁₋₅-alkyl)₂; or
a 5- to 10-membered aryl or heteroaryl radical, which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may contain 1, 2 or 3 heteroatom(s) independently selected from the group consisting of nitrogen, oxygen and sulfur as chain member(s) and which may be unsubstituted or substituted with 1, 2 or 3 substituent(s) independently selected from the group consisting of F, Cl, Br, I, - CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂ and whereby said heteroaryl radical contains 1, 2 or 3 heteroatom(s) independently selected from the group consisting of nitrogen, oxygen and sulfur as ring member(s);
R² represents a hydrogen atom or a -C(=O)-R¹⁰ moiety;
or
R¹ and R² together with the bridging nitrogen form a Nitro (NO₂)-group or
a 5- or 6-membered heteroaryl radical which may contain 1, 2 or 3 additional heteroatom(s) independently selected from the group consisting of nitrogen, oxygen and sulfur as ring member(s) and which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-Phenyl, phenyl, phenoxy and benzyl and which may be condensed with a monocyclic ring system which may be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
whereby the ring of the ring system is 5- 6- or 7-membered and may contain 1, 2 or 3 heteroatom(s) independently selected from the group consisting of nitrogen, oxygen and sulfur;
R³ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
R⁴ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
R⁵ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; or
a 5- to 10-membered aryl or heteroaryl radical which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
R⁶ represents a hydrogen atom or
a 5- to 10-membered aryl or heteroaryl radical which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
R⁷ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
R⁸ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; or
a 5- to 10-membered aryl or heteroaryl radical which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl,--C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂ and whereby the heteroaryl radical contains 1, 2 or 3 heteroatom(s) independently selected from the group consisting of nitrogen, oxygen and sulfur as ring member(s);
R⁹ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; or
a 5- to 10-membered aryl radical which may be unsubstituted or substituted with 1, 2 or 3 substituent(s) independently selected from the group consisting of C₁₋₅-alkyl, -0-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), - C(=O)-N(C₁₋₅-alkyl)₂, -S(=O)₂-C₁₋₅-alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, - CF₃, -OCF₃, -SCF3, -OH, -SH, -NH₂, -O-C₁₋₅-alkyl, -S-C₁₋₅-alkyl, -NH(C₁₋₅-alkyl) and - N(C₁₋₅-alkyl)₂;
and
R¹⁰ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferably one or both of the following provisos may apply to any of the definitions given herein, namely
that R¹ and R² do not both represent a hydrogen atom if X represents an -NH₂ group and
that R¹ does not represent a hydrogen atom if X represents an -NH-S(=O)₂₋R⁸ group.

Preferred are substituted phenyl-piperazine compounds of general formula I given above, wherein R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, -(CH₂)-(CH₂)-, -(CH₂)-(CH₂)-(CH₂)-, -O-(CH₂)-(CH₂)- or - (CH₂)-(CH₂)-O-group and/or may be unsubstituted or substituted with 1, 2 or 3 substituent(s) independently selected from the group consisting of C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C1-₅-alkyl)₂, -S(=O)₂-C₁₋₅-alkyl, -S(=O)₂-phenyl, phenyl, phenoxy and benzyl;
preferably R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)₁, ₂ or ₃- group and/or may be unsubstituted or substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, - CN, -CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, -(CH₂)-(CH₂)-, -(CH₂)-(CH₂)-(CH₂)-, -O-(CH₂)-(CH₂)- or - (CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=C)-C₂H₅, - C(=O)-CH₂-CH₂-CH₃, -C(=O)-CH(CH₃)₂, -C(=O)-C(CH₃)₃, F, Cl, Br, I, -ON, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
more preferably R¹ represents
a hydrogen atom; or
an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂₋CH₂-CH₃, -O-CH(CH₃)₂, -O-C(CH₃)₃, F, Cl, Br, -CN, -CF₃, -OCF₃, -OH and -SH;
and X and R² to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R¹ and R² together with the bridging nitrogen atom form a nitro group; or a moiety selected from the group consisting of 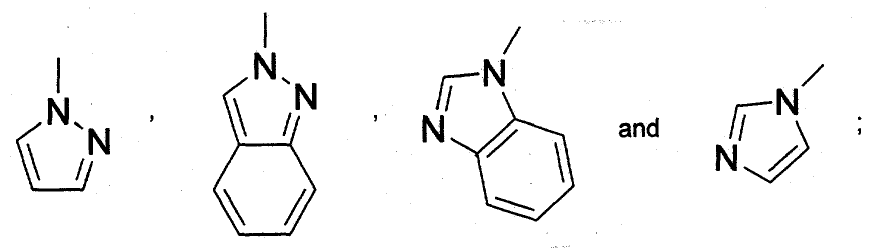 whereby each of these afore mentioned cyclic moieties may be unsubstituted substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹ and R² together with the bridging nitrogen atom form a nitro group or a moiety selected from the group consisting of 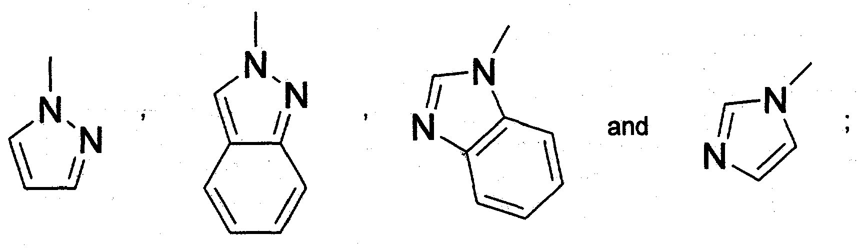 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,
more preferably R¹ and R² together with the bridging nitrogen atom form a nitro group or a moiety selected from the group consisting of 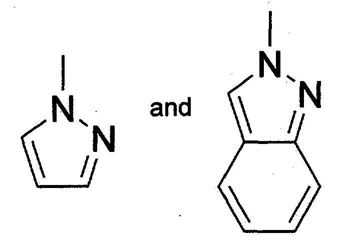 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,
and X and R³ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R³ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, - SCF3, -OH and -SH;
preferably R³ represents an alkyl radical selected from the group consisting of methyl, ethyl, n-propyl, isopropyl, n-butyl, sec-butyl, isobutyl and tert-butyl;
more preferably R³ represents a methyl or ethyl radical,
and X, R¹, R² and R⁴ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁴ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, - SCF₃, -OH and -SH;
preferably R⁴ represents an alkyl radical selected from the group consisting of methyl, ethyl, n-propyl, isopropyl, n-butyl, sec-butyl, isobutyl and tert-butyl;
more preferably R⁴ represents a methyl or ethyl radical,
and X, R¹ to R³ and R⁵ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁵ represents
a linear or branched C₁₋₁₀ alkyl radical, which may be unsubstituted or substituted with 1, 2 or 3 substituent(s) independently selected from the group consisting of -0-C₁₋₅-alkyl, -S-C₁₋₅-alkyl, F, Cl, Br, I, -CN, -CF₃, -OCF₃, -SCF₃, -OH and SH;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁵ represents
an alkyl radical selected from the group consisting of methyl, ethyl, n-propyl, isopropyl, n-butyl, sec-butyl, isobutyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
more preferably R⁵ represents
an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂₋CH₂-CH₃, -O-CH(CH₃)₂, -O-C(CH₃)₃, F, Cl, Br, -CN, -CF₃, -OCF₃, -OH and -SH,
and X, R¹ to R⁴ and R⁶ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁶ represents
a hydrogen atom or
an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, 1, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂; -S(=O)₂-C₁₋₅-alkyl, -S(=O)₂-phenyl, phenyl, phenoxy and benzyl;
preferably R⁶ represents
a hydrogen atom or
an aryl radical selected from the group consisting of phenyl and naphthyl, whereby said aryl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, - O-CH₃, -O-C₂H₅, F, Cl, Br, -CF₃, -OCF₃, -OH and -SH;
more preferably R⁶ represents a hydrogen atom, or
a phenyl radical, whereby said phenyl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br,-CF₃, -OCF₃, -OH and -SH,
and X, R¹ to R⁵ and R⁷ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁷ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, - SCF₃, -OH and -SH;
preferably R⁷ represents an alkyl radical selected from the group consisting of methyl, ethyl, n-propyl, isopropyl, n-butyl, sec-butyl, isobutyl and tert-butyl;
more preferably R⁷ represents a methyl or ethyl radical,
and X, R¹ to R⁶ and R⁸ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁸ represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁸ represents
an alkyl radical selected from the group consisting of methyl, ethyl, n-propyl, isopropyl, n-butyl, sec-butyl, isobutyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1,2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
more preferably R⁸ represents
an alkyl radical selected from the group consisting of methyl, ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH₂)-, or -(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, - CN, -CF₃, -OCF₃, -OH and -SH,
and X, R¹ to R⁷, R⁹ and R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁹ represents
a linear or branched C₁₋₁₀ alkyl radical, which may be unsubstituted or substituted with 1, 2 or 3 substituent(s) independently selected from the group consisting of -0-C₁₋₅-alkyl, -S-C₁₋₅-alkyl, F, Cl, Br, I, -CN, -CF₃, -OCF₃, -SCF₃, -OH and SH;
or an aryl radical selected from the group consisting of phenyl and naphthyl, whereby said aryl radical may be bonded via a -(CH₂)_{1,2} or ₃-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁹ represents
an alkyl radical selected from the group consisting of methyl, ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
more preferably R⁹ represents
an alkyl radical selected from the group consisting of methyl, ethyl, n-propyl, isopropyl, n-butyl, sec-butyl, isobutyl and tert-butyl;
or an aryl radical selected from the group consisting of phenyl and naphthyl, whereby said aryl radical may be bonded via a -(CH₂)-, -(CH₂)-(CH₂)- or -(CH₂)-(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -0-CH(CH₃)₂, -O-C(CH₃)₃, F, Cl, Br, -CN, -CF₃, -OCF₃, -OH and -SH,
and X, R¹ to R⁸ and R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R¹⁰ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, - SCF₃, -OH and -SH;
preferably R¹⁰ represents an alkyl radical selected from the group consisting of methyl, ethyl, n-propyl, isopropyl, n-butyl, sec-butyl, isobutyl and tert-butyl;
more preferably R¹⁰ represents a methyl or ethyl radical,
and X and R¹ to R⁹ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are substituted phenyl-piperazine compounds of general formula I given above, wherein
X represents -CN, -C(=O)-OH, -C(=O)-OR⁴, -O-R⁵, -NH₂, -NR⁶-O(=O)-R⁷, -NH-S(=O)₂-R⁸ or -N-H-R⁹;
R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)₁; _{2 or 3}- group and/or may be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, -(CH₂)-(CH₂)-, -(CH₂)-(CH₂)-(CH₂)-, -O-(CH₂)-(CH₂)- or - (CH₂)-(CH₂)-O-group and/or may be unsubstituted or substituted with 1, 2 or 3 substituent(s) independently selected from the group consisting of C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R² represents a hydrogen atom or a -C(=O)-R¹⁰ moiety;
R¹ and R² together with the bridging nitrogen atom form a nitro group; or a moiety selected from the group consisting of 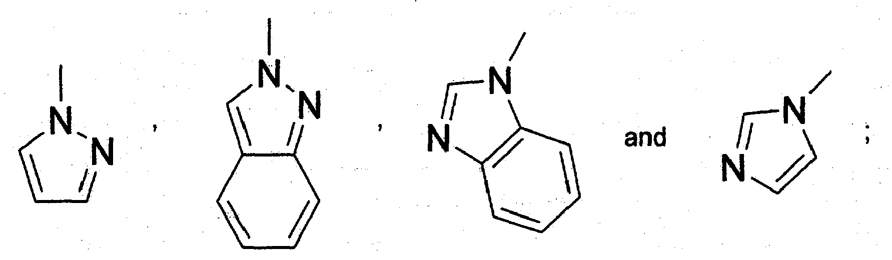 whereby each of these afore mentioned cyclic moieties may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R³ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and - SH;
R⁴ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and - SH;
R⁵ represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃,-OCF₃, -SCF₃, -OH, -SH, -NH₂, -NH(C₁₋₅₋alkyl), -N(C₁₋₅-alkyl)₂, -NO₂, -CHO, -CF₂H,-CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁶ represents
a hydrogen atom or
an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁷ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and - SH;
R⁸ represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH; or
an aryl or heteroaryl radical selected from the group consisting of phenyl, naphthyl, furyl (furanyl), thienyl (thiophenyl), pyrrolyl, oxazolyl, isoxazolyl, thiazoiyl, isothiazolyl, imidazolyl, pyrazolyl, oxadiazolyl, thiadiazolyl, triazolyl, pyridinyl, pyrrdazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2} or ₃-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
**R**^{**9**} **represents**
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH;
or an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, CI, Br, I, -CN, CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R¹⁰ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and - SH,
optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt-thereof, or a corresponding solvate thereof.

Particularly preferred are also substituted phenyl-piperazine compounds of general formula I given above, wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, - CN, -CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, -(CH₂)-(CH₂)-, -(CH₂)-(CH₂)-(CH₂)-, -O-(CH₂)-(CH₂)- or - (CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂₋CH₂-CH₃, -C(=O)-O-CH(CH₃)₂, -C(=O)-O-C(CH₃)₃, -C(=O)-CH₃, -C(=O)-C₂H₅, - C(=O)-CH₂-CH₂-CH₃, -C(=O)-CH(CH₃)₂, -C(=O)=C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
R² represents a hydrogen atom or a -C(=O)-R¹⁰ moiety;
preferably R¹ and R² together with the bridging nitrogen atom form a nitro group or a moiety selected from the group consisting of 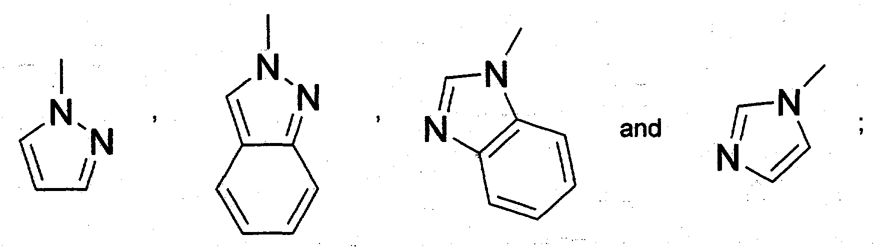 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,
R³ represents an alkyl radical selected from the group consisting of methyl, ethyl, n-propyl, isopropyl, n-butyl, sec-butyl, isobutyl and tert-butyl;
R⁴ represents an alkyl radical selected from the group consisting of methyl, ethyl, n-propyl, isopropyl, n-butyl, sec-butyl, isobutyl and tert-butyl;
R⁵ represents
an alkyl radical selected from the group consisting of methyl, ethyl, n-propyl, isopropyl, n-butyl, sec-butyl, isobutyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁶ represents
a hydrogen atom or
an aryl radical selected from the group consisting of phenyl and naphthyl, whereby said aryl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, - O-CH₃, -O-C₂H₅, F, Cl, Br,-CF₃, -OCF₃, -OH and -SH,
R⁷ represents an alkyl radical selected from the group consisting of methyl, ethyl, n-propyl, isopropyl, n-butyl, sec-butyl, isobutyl and fert-butyl;
R⁸ represents
an alkyl radical selected from the group consisting of methyl, ethyl, n-propyl, isopropyl, n-butyl, sec-butyl, isobutyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2} or ₃-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁹ represents
an alkyl radical selected from the group consisting of methyl, ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH2)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R¹⁰ represents an alkyl radical selected from the group consisting of methyl, ethyl, n-propyl, isopropyl, n-butyl, sec-butyl, isobutyl and tert-butyl,
optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Particularly preferred are also substituted phenyl-piperazine compounds of general formula I given above, wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents
a hydrogen atom; or
an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂₋CH₂-CH₃, -O-CH(CH₃)₂, -O-C(CH₃)₃, F, Cl, Br, -CN, -CF₃, -OCF₃, -OH and -SH. R² represents a hydrogen atom or a -C(=O)-R¹⁰ moiety;
R¹ and R² together with the bridging nitrogen atom form a nitro group or moiety selected from the group consisting of 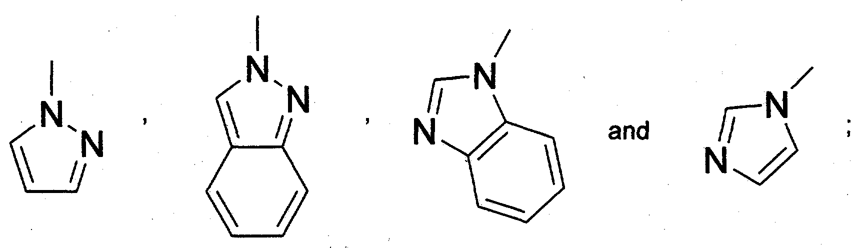 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,
R³ represents a methyl or ethyl radical;
R⁴ represents a methyl or ethyl radical;
R⁵ represents
an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)- or -(CH₂)-(CH₂)-(CH₂)- group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, - CN, -CF₃, -OCF₃, -OH and -SH;
R⁶ represents a hydrogen atom, or
a phenyl radical, whereby said phenyl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br, -CF₃, -OCF₃, -OH and -SH;
R⁷ represents a methyl or ethyl radical;
R⁸ represents
an alkyl radical selected from the group consisting of methyl, ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH₂)-, or -(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, - CN, -CF₃, -OCF₃, -OH and -SH,
R⁹ represents
an alkyl radical selected from the group consisting of methyl, ethyl, n-propyl, isopropyl, n-butyl, sec-butyl, isobutyl and tert-butyl;
or an aryl radical selected from the group consisting of phenyl and naphthyl, whereby said aryl radical may be bonded via a -(CH₂)-, -(CH₂)-(CH₂)- or -(CH₂)-(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH;
R¹⁰ represents a methyl or ethyl radical,
optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Particularly preferred are also substituted phenyl-piperazine compounds of general formula I given above, wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents
a hydrogen atom; or
an unsubstituted aryl or heteroaryl radical selected from the group consisting of phenyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or - (CH₂)-(CH₂)-O=group,
R² represents a hydrogen atom or a -C(=O)-R¹⁰ moiety;
or
R¹ and R² together with the bridging nitrogen atom form a nitro group or a cyclic moiety of the following formula: 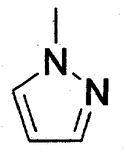 whereby said cyclic moiety may be substituted with 1, 2 or 3 substituent(s) independently selected from the group consisting of methyl, ethyl, n-propyl, isopropyl, n-butyl, isobutyl, sec-butyl and tert-butyl;
R³ represents a methyl or ethyl radical;
R4 represents a methyl or ethyl radical;
R⁵ represents a methyl radical, an ethyl radical; or an unsubstituted phenyl radical, which may be bonded via a -(CH₂)-group;
R⁶ represents a hydrogen atom or an unsubstituted benzyl radical;
R⁷ represents a methyl or ethyl radical;
R⁸ represents a methyl or ethyl radical; or an unsubstituted phenyl radical;
R⁹ represents an unsubstituted benzyl radical;
and
R¹⁰ represents a methyl or ethyl radical,
optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

Most preferred are substituted phenyl-piperazine compounds of general formula I given above selected from the group consisting of
[1] 4-(4-Methyl-piperazin-1-yl)-2-phenethylamino-benzoic acid,
[2] 2-[(Furan-2-ylmethyl)-amino]-4-(4-methyl-piperazin-1-yl)-benzonitrile,
[3] 2-[(Furan-2-ylmethyl)-amino]-4-(4-methyl-piperazin-1-yl)-benzoic acid,
[4] 2-Benzylamino-4-(4-methyl-piperazin-1-yl)-benzoic acid methyl ester,
[5] 2-Benzylamino-4-(4-methyl-piperazin-1-yl)-benzonitrile,
[6] 4-(4-Methyl-piperazin-1-yl)-2-phenethylamino-benzoic acid methyl ester,
[7] 2-[(Furan-2-ylmethyl)-amino]-4-(4-methyl-piperazin-1-yl)-benzoic acid methyl ester,
[8] 2-Benzylamino-4-(4-methyl-piperazin-1-yl)-benzoic acid,
[9] [2-Benzyloxy-5-(4-methyl-piperazin-1-yl)-phenyl]-phenethyl-amine,
[10] [2-Benzyloxy-5-(4-methyl-piperazin-1-yl)-phenyl]-furan-2-yl-methyl amine,
[11] Benzyl-[2-methoxy-5-(4-methyl-piperazin-1-yl)-phenyl]-amine,
[12] [2-Methoxy-5-(4-methyl-piperazin-1-yl)-phenyl]-phenethyl-amine,
[13] Furan-2-ylmethyl-[2-methoxy-5-(4-methyl-piperazin-1-yl)-phenyl]-amine,
[14] Benzyl-(2-benzyloxy-5-(4-methyl-piperazin-1-yl)-phenyl]-amine,
[15] N-[2-Acetyl-(2-phenoxyethyl)-amino]-4-(4-methyl-piperazin-1-yl)-phenyl]-acetamide,
[16] N-[4-(4-Methyl-piperazin-1-yl)-2-(2-phenoxy-ethylamino)-phenyl]-acetamide,
[17] N-[2-(Acetyl-amino)-4-(4-methyl-piperazin-1-yl)-phenyl]-N-benzyl-acetamide,
[18] N-[2-(3,5-Dimethyl-pyrazol-1-yl)-4-(4-methyl-piperazin-1-yl)-phenyl]-acetamide,
[19] N-[2-(Acetyl-furan-2-ylmethyl-amino)-4-(4-methyl-piparazin-1-yl)-phenyl]-acetamide,
[20] N-[2-Benzylamino-4-(4-methyl-piperazin-1-yl)-phertyl]-acetamide,
[21] N-[2-[Furan-2-ylmethyl)-amino]-4-(4-methyl-piperazin-1-yl)-phenyl]-acetamide,
[22] N-[2-Amino-5-(4-methyl-piperazin-1-yl)-phenyl]-N-furan-2-ylmethyl-acetamide,
[23] N-[2-Amino-4-(4-methyl-piperazin-1-yl)-phenyl]-N-benzyl-acetamide,
[24] N-[2-Benzylamino-4-(4-methyl-piperazin-1-yl)-phenyl]-benzenesulfonamide,
[25] N-[2-Benzylamino-4-(4-methyl-piperazin-1-yl)-phenyl]-methansulfonamide,
[26] 2-Benzyloxy-5-(4-methyl-piperazin-1-yl)-phenylamine,
[27] Benzyl-[4-(4-methyl-piperazin-1-yl)-2-nitro-phenyl]-amine and
[28] 2-Cyano-(5-piperazin-1-yl-methyl)-2-phenoxy-ethylamine
   optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

In yet another aspect the present invention relates tosubstituted phenyl-piperazine compounds of general formula I, 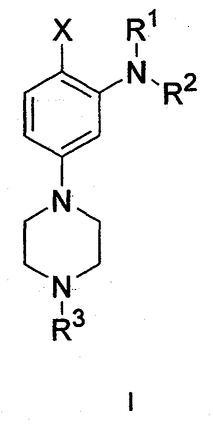 wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents a hydrogen atom; a saturated or unsaturated, optionally at least mono-substituted, optionally at least one heteroatom as a ring member containing cycloaliphatic radical, which is bonded via a linear or branched, optionally at least mono-substituted alkylene, alkenylene or alkinylene group; or an optionally at least mono-substituted aryl or heteroaryl radical, which is bonded via a linear or branched, optionally at least mono-substituted, optionally at least one heteroatom as a chain member containing alkylene, alkenylene or alkinylene group;
R² represents a hydrogen atom or a -C(=O)-R¹⁰ moiety;
or
R¹ and R² together with the bridging nitrogen form a nitro (NO₂)-group; an optionally at least mono-substituted heteroaryl radical which may contain at least one further heteroatom as a ring member and/or which may be condensed with an optionally at least mono-substituted monocyclic ring system;
R³ represents a linear or branched, saturated or unsaturated, optionally at least mono-substituted aliphatic radical;
R⁴ represents a linear or branched, saturated or unsaturated, optionally at least mono-substituted aliphatic radical;
R⁵ represents a linear or branched, saturated or unsaturated, optionally at least mono-substituted aliphatic radical; or an optionally at least mono-substituted aryl or heteroaryl radical, which may be bonded via a linear or branched, optionally at least mono-substituted alkylene, alkenylene or alkinylene group;
R⁶ represents a hydrogen atom or an optionally at least mono-substituted aryl radical, which may be bonded via a linear or branched, optionally at least mono-substituted alkylene, alkenylene or alkinylene group;
R⁷ represents a linear or branched, saturated or unsaturated, optionally at least mono-substituted aliphatic radical;
R⁸ represents a linear or branched, saturated or unsaturated, optionally at least mono-substituted aliphatic radical; or an optionally at least mono-substituted aryl or heteroaryl radical, which may be bonded via a linear or branched, optionally at least mono-substituted alkylene, alkenylene or alkinylene group;
R⁹ represents a linear or branched, saturated or unsaturated, optionally at least mono-substituted aliphatic radical; or an optionally at least mono-substituted aryl radical, which may be bonded via a linear or branched, optionally at least mono-substituted alkylene, alkenylene or alkinylene group;
and
R¹⁰ represents a linear or branched, saturated or unsaturated, optionally at least mono-substituted aliphatic radical;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

If any of the substituents represents or comprises a (hetero)cycloaliphatic radical [(hetero)cycloaliphatic group], said (hetero)cycloaliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of oxo (=O), thia (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH2, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂C₁₋₅-alkyl, -S(=O)₂-phenyl, phenyl, phenoxy and benzyl; whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, - SCF₃, -OH, -SH, -NH₂ and -NO₂.

More preferably said substituents may be selected independently from the group consisting of oxo (=O), thia (=S), methyl, ethyl, n-propyl, iso-propyl, n-butyl, tert-butyl, sec-butyl, iso-butyl, n-pentyl, -O-CH₃, -O-C₂H₅, -O-CH₂-CH₂-CH₃, -O-CH(CH₃)₂, -0-C(CH₃)₃, -S-CH₃, -S-C₂H₅, -S-CH₂-CH₂-CH₃, -S-CH(CH₃)₂, -S-C(CH₃)₃, -O(=O)-OH, - C(=O)-O-CH₃, -C(=O)-O-C₂H₅, -C(=O)-O-CH₃-CH₃-CH₃, -C(=O)-O-CH(CH₃)₂, - C(=O)-O-C(CH₃)₃, -C(=O)-CH₃, -C(=O)-C₂H₅, -C(=O)-CH₃-CH₃₋CH₃, -C(=O)-CH(CH₃)₂, -C(=O)-C(CH₃)₃, F, Cl, Br, I, -CN, -CF₃, -OCF₃, -SCF₃, -OH, -SH, -NH₂, - NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂, -N02, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurence said cyclic substituents may be unsubstituted or substituted by 1, 2 or 3 substituent(s) independently selected from the group consisting of methyl, ethyl, n-propyl, iso-propyl, methoxy, ethoxy, F, Cl, Br,-CN, -CF₃, -OCF₃, -SCF₃, -OH, -SH, -NH₂ and -N0₂.

If any of the substituents represents or comprises a cycloaliphatic radical, which contains one or more, preferably 1, 2 or 3 heteroatom(s) as ring member(s), unless defined otherwise, each of these heteroatom(s) may preferably be selected independently from the group consisting of N, O and S.

Suitable (hetero)cycloaliphatic radicals, which may be unsubstituted or at least mono-substituted, include cyclopropyl, cyclobutyl, cyclopentyl, cyclopentenyl, cyclohexyl, cyclohexenyl, cycloheptyl, cycloheptenyl, cyclooctyl, cyclooctenyl, cyclononyl, imidazolidinyl, aziridinyl, azetidinyl, pyrrolidinyl, piperidinyl, morpholinyl, thiomorpholinyl, piperazinyl, pyrazolidinyl, tetrahydrofuranyl, tetrahydrothiophenyl, diazepanyl and azepanyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of -O-C₁₋₅-alkyl, -S-C₁₋₅-alkyl, -F, Cl, Br, I, -CN, -CF₃, -OCF₃, -SCF₃, -OH, -SH, -NH₂, -NH(C₁₋₅-alkyl), -N(C₁₋₅-alkyl)₂ and phenyl, whereby in each occurence C₁₋₅-alkyl may be linear or branched and the phenyl radical is preferably unsubstituted. An alkenylene group comprises at least one carbon-carbon double bond, an alkinylene group comprises at least one carbon-carbon triple bond.

Suitable alkylene groups include -(CH₂)-, -CH(CH₃)-, -CH(phenyl), -(CH₂)₂-, -(CH₂)₃₋,-(CH₂)₄-,-(CH₂)₅ and -(CH₂)₆-, suitable alkenylene groups include -CH=CH-, -CH₂₋CH=CH- and -CH=CH-CH₂- and suitable alkinylene groups include -C**≡**C-, -CH₂₋C≡C- and -C≡C-CH₂-.

If the alkylene, alkenylene or alkinylene group contains one or more, preferably 1, 2 or 3, more preferably 1, heteroatom(s) as chain member(s), unless defined otherwise, each of these heteroatom(s) may preferably be selected from the group consisting of N, 0 and S. Suitable alkylene groups which contain one or more heteroatom(s) include -CH₂-O-CH₂- and -CH₂-CH₂-O.

If any of the substituents represents or comprises an aryl radical (aryl group), including a phenyl or naphthyl group, said aryl radical may - if not defined otherwise-be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅₋alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -SH, -NH₂, -NH(C₁₋₅-alkyl), -N(C₁₋₅-alkyl)₂, -NO₂,-CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl; whereby in each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and - NO₂.

More preferably said substituents may be selected independently from the group consisting of methyl, ethyl, n-propyl, iso-propyl, n-butyl, tert-butyl, sec-butyl, iso-butyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₃-CH₃-CH₃, -C(=O)-O-CH(CH₃)₂, -C(=O)-O-C(CH₃)₃, - C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

Preferred aryl radicals, which may optionally be at least mono-substituted, are phenyl and naphthyl.

If any of the substituents represents or comprises a heteroaryl radical (heteroaryl group), said heteroaryl radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl; whereby in each occurrence C₁₋₅₋alkyl may be linear or branched and whereby said cyclic substituent(s) may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and -NO₂.

More preferably said substituents may be selected independently from the group consisting of methyl, ethyl, n-propyl, iso-propyl, n-butyl, tert-butyl, sec-butyl, iso-butyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₃-CH₃-CH₃, -C(=O)-O-CH(CH₃)₂, -C(=O)-O-C(CH₃)₃, - C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃.

The heteroatom(s), which are present as ring member(s) in the heteroaryl radical, may, unless defined otherwise, independently be selected from the group consisting of nitrogen, oxygen and sulphur. Preferably the heteroaryl radical comprises 1, 2 or 3 heteroatom(s).

Suitable heteroaryl radicals, which may optionally be at least mono-substituted, may preferably be selected from the group consisting of furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl, benzoxadiazolyl, benzoxazolyl, benzthiazolyl, benzisoxazolyl, benzisothiazolyl and imidazo[2,1-b]thiazolyl.

If at least two of the substituents together with the bridging nitrogen atom form an optionally at least mono-subsituted heteroaryl radical which may be condensed with an optionally at least mono-substituted mono- or bicyclic ring system, said heteroaryl radical may preferably be selected from the group consisting of

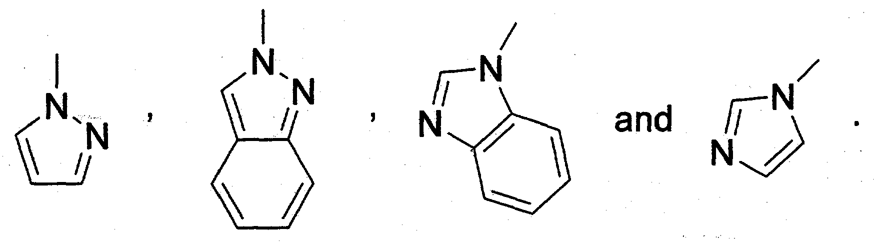

Said heteroaryl radical may - if not defined otherwise - be unsubstituted or substituted by one or more substituents , preferably unsubstituted or substituted with 1, 2, 3, 4 or 5 substituent(s). Said substituent(s) may preferably be selected independently from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, - CN, -CF₃, -OCF₃, -SCF₃, -OH, -SH, -NH₂, -NH(C₁₋₅-alkyl), -N(C₁₋₅-alkyl)₂, -NO₂, - CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl; whereby in Each occurrence C₁₋₅-alkyl may be linear or branched and whereby said cyclic substituent(s) may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and - NO₂.

More preferably said substituents my be selected from the group consisting of methyl, ethyl, n-propyl, iso-propyl, n-butyl, tert-butyl, sec-butyl, iso-butyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, - C(=O)-C₂H₅, -C(=O)-CH₃-CH₃-CH₃, -C(=O)-CH(CH₃)₂, -C(=O)-C(CH₃)₃, F, Cl, Br, I, - CN, -CF₃, -OCF₃, -SCF₃, -OH, -SH, -NH₂, -NH-CH₃, -NH-C₂H₅, -NH-CH₂-CH₂-CH₃, - NH-CH(CH₃)₂, -NH-C(CH₃)₃, -N(CH₃)₂, -N(C₂H₅)₂, -NO₂, -CHO, -CF₂H, -CFH₂, - C(=O)-NH₂, -C(=O)-NH-CH₃, -C(=O)-NH-C₂H₅, -C(=O)-N(CH₃)₂, -C(=O)-N(C₂H₅)₂, - S(=O)₂-CH₃, -S(=O)₂-phenyl, phenyl, phenoxy and benzyl.

A mono- or bicyclic ring system according to the present invention - if not defined otherwise - means a mono- or bicyclic hydrocarbon ring system that may be saturated, unsaturated or aromatic. Each of its different rings may show a different degree of saturation, i.e. it may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of N, 0 and S. The rings of the mono- or bicyclic ring system are preferably 5-, 6- or 7-membered.

Preferably a mono-or bicyclic ring system according to the present invention is a phenyl or naphthyl ring system.

The term "condensed" according to the present invention means that a ring or ring system is attached to another ring or ring system, whereby the terms "annulated" or "annelated" are also used by those skilled in the art to designate this kind of attachment.

Such a mono- or bicyclic ring system may - if not defined otherwise - be unsubstituted or substituted by one or more substituents, preferably unsubstituted or substituted with 1, 2, 3, 4 or 5 substituent(s). Said substituents may preferably be selected independently from the group consisting of C₁₋₅-alkyl, -O-C₁₋₅-alkyl, -S-C₁₋₅₋alkyl, -C(=O)-OH, oxo (=O), thia (=S), -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl; whereby in each occurence C₁₋₅-alkyl may be linear or branched and whereby said cyclic substituents may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and -NO₂.

More preferably said substituents may be selected from the group consisting of oxo (=O), thia (=S), methyl, ethyl, n-propyl, iso-propyl, n-butyl, tert-butyl, sec-butyl, iso-butyl, n-pentyl, -O-CH₃, -O-C₂H₅, -O-CH₂-CH₂-CH₃, -O-CH(CH₃)₂, -O-O(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃-CH₃-CH₃, -C(=O)-O-CH(CH₃)₂, -C(=O)-O-C(CH₃)₃, -C(=O)-CH₃, -C(=O)-C₂H₅, -C(=O)-CH₃-CH₃-CH₃, -C(=O)-CH(CH₃)₂, - C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, - CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, -S(=O)₂-phenyl, phenyl, phenoxy and benzyl; whereby in each occurence said cyclic substituents may be unsubstituted or substituted by 1, 2 or 3 substituent(s) independently selected from the group consisting of methyl, ethyl, n-propyl, iso-propyl, methoxy, ethoxy, F, Cl, Br, -CN, -CF₃, -OCF₃, -SCF₃, -OH, -SH, -NH₂ and -NO₂.

If any of the substituents represents a saturated or unsaturated aliphatic radical (aliphatic group), i.e. an alkyl radical, an alkenyl radical or an alkinyl radical, said aliphatic radical may - if not defined otherwise - be unsubstituted or substituted by one or more substituents, preferably unsubstituted or substituted with 1, 2, 3, 4 or 5 substituent(s). Said substituent(s) may preferably be selected independently from the group consisting of -O-C₁₋₅-alkyl, -S-C₁₋₅-alkyl, F, Cl, Br, I, -CN, -CF₃, -OCF₃, -SCF₃, - OH, -SH, -NH₂, -NH(C₁₋₅-alkyl) and -N(C₁₋₅-alkyl)₂ , whereby in each occurence C₁₋₅₋alkyl may be linear or branched. More preferably said substituent(s) may preferably be selected independently from the group consisting of =O-CH₃, -O-C₂H₅, -O-CH₂₋CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, F, Cl, Br, I, -CN, -CF₃, -OCF₃, -SCF₃, -OH, -SH, -NH₂, NH-CH₃, -NH-C₂H₅, -NH-CH₂-CH₂-CH₃, -NH-CH(CH₃)₂, -NH-C(CH₃)₃, -N(CH₃)₂, - N(C₂H₅)₂.

An alkenyl radical comprises at least one carbon-carbon double bond, an alkinyl radical comprises at least one carbon-carbon triple bond.
Suitable alkyl radicals, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl and n-decyl.

Suitable alkenyl radicals, which may be substituted by one or more substituents, may preferably be selected from the group consisting of vinyl, 1-propenyl, 2-propenyl, 1-butenyl, 2-butenyl and 3-butenyl.

Suitable alkinyl radicals, which may be substituted by one or more substituents, may preferably be selected from the group consisting of ethinyl, 1-propinyl, 2-propinyl, 1-butinyl, 2-butinyl and 3-butinyl.

Preferred are compounds of general formula I given above, wherein
X represents -CN, -C(=O)-OH, -C(=O)-OR⁴, -O-R⁵, -NH₂, -NR⁶-C(=O)-R⁷, -NH-S(=O)₂-R⁸; or -NH-R⁹;
R¹ represents a hydrogen atom; a saturated or unsaturated, optionally at least mono-substituted 3- to 9-membered cycloaliphatic radical, which is bonded via a linear or branched, optionally at least mono-substituted C₁₋₆ alkylene, C₂₋₆ alkenylene or C₂₋₆ alkinylene group and/or which may contain 1, 2 or 3 heteroatom(s) independently selected from the group consisting of nitrogen, oxygen and sulfur as ring member(s); or an optionally at least mono-substituted 5- to 10-membered aryl or heteroaryl radical, which is bonded via a linear or branched, optionally at least mono-substituted C₁₋₆ alkylene, C₂₋₆ alkenylene or C₂₋₆ alkinylene group which may contain 1, 2 or 3 heteroatom(s) independently selected from the group consisting of nitrogen, oxygen and sulfur as chain member(s) and whereby the heteroaryl radical contains 1, 2 or 3 heteroatom(s) independently selected from the group consisting of nitrogen, oxygen and sulfur as ring member(s);
R² represents a hydrogen atom or a -C(=O)-R¹⁰ moiety;
or
R¹ and R² together with the bridging nitrogen form a Nitro (NO₂)-group or an optionally at least mono-substituted 5- or 6-membered heteroaryl radical which may contain 1, 2 or 3 additional heteroatom(s) independently selected from the group consisting of nitrogen, oxygen and sulfur as ring member(s) and/or which may be condensed with an optionally at least mono-substituted monocyclic ring system;
whereby the ring of the ring system is 5- 6- or 7-membered and may contain 1, 2 or 3 heteroatom(s) independently selected from the group consisting of nitrogen, oxygen and sulfur;
R³ represents a linear or branched, saturated or unsaturated, optionally at least mono-substituted C₁₋₁₀ aliphatic radical;
R⁴ represents a linear or branched, saturated or unsaturated, optionally at least mono-substituted C₁₋₁₀ aliphatic radical;
R⁵ represents a linear or branched, saturated or unsaturated, optionally at least mono-substituted C₁₋₁₀ aliphatic radical; or an optionally at least mono-substituted 5-to 10-membered aryl or heteroaryl radical which maybe bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulfur as ring member(s);
R⁶ represents a hydrogen atom or an optionally at least mono-substituted 5- to 10-membered aryl or heteroaryl radical which may be bonded via a linear or branched, optionally at least mono-substituted C₁₋₆ alkylene, C₂₋₆ alkenylene or C₂₋₆ alkinylene group;
R⁷ represents a linear or branched, saturated or unsaturated, optionally at least mono-substituted C₁₋₁₀ aliphatic radical;
R⁸ represents a linear or branched, saturated or unsaturated, optionally at least mono-substituted C₁₋₁₀ aliphatic radical; or an optionally at least mono-substituted 5-to 10-membered aryl or heteroaryl radical which may be bonded via a linear or branched, optionally at least mono-substituted C₁₋₆ alkylene, C₂₋₆ alkenylene or C₂₋₆ alkinylene group and whereby the heteroaryl radical contains 1, 2 or 3 heteroatom(s) independently selected from the group consisting of nitrogen, oxygen and sulfur as ring member(s);
R⁹ represents a linear or branched, saturated or unsaturated, optionally at least mono-substituted C₁₋₁₀ aliphatic radical; or an optionally at least mono-substituted 5-to 10-membered aryl radical which may be bonded via a linear or branched, optionally at least mono-substituted C₁₋₆ alkylene, C₂₋₆ alkenylene or C₂₋₆ alkinylene group;
and
R¹⁰ represents a linear or branched, saturated or unsaturated, optionally at least mono-substituted C₁₋₁₀ aliphatic radical.

Preferred are also substituted phenyl-piperazine compounds of general formula I given above, wherein R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thia (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazoiyl, whereby said aryl or heteroaryl radical is bonded via a -(CH₂)-, -(CH₂)-(CH₂)-, -(CH₂)-(CH₂)-(CH₂)-, -O-(CH₂)-(CH₂)- or - (CH₂)-(CH₂)-O-group and/or may be unsubstituted or substituted with 1, 2 or 3 substituent(s) independently selected from the group consisting of C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thia (=S), methyl, ethyl, n-propyl, iso-propyl, n-butyl, tert-butyl, sec-butyl, iso-butyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃,-C(=O)-O-C₂H₅, -C(=O)-O-CH₃-CH₃-CH₃, -C(=O)-O-CH(CH₃)₂, -C(=O)-O-C(CH₃)₃, - C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-GH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazihyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, -(CH₂)-(CH₂)-, -(CH₂)-(CH₂)-(CH₂)-, O-(CH₂)-(CH₂)- or - (CH₂)-(CH₂)-O-group and/or may be unsubstituted or substituted with 1, 2 or 3 substituent(s) independently selected from the group consisting of methyl, ethyl, n-propyl, iso-propyl, n-butyl, tert-butyl, sec-butyl, iso-butyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃₋CH₃-CH₃, -C(=O)-O-CH(CH₃)₂, -C(=O)-O-C(CH₃)₃, -C(=O)-CH₃, -C(=O)-C₂H₅,-C(=O)-CH₃-CH₃-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
more preferably R¹ represents
a hydrogen atom; or
an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O(CH₂)-(CH₂)-, or -(CH₂)-(CH₂)-O-group and/or may unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH,
and X and R² to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R¹ and R² together with the bridging nitrogen atom form a nitro group; or an optionally at least mono-substituted moiety selected from the group consisting of 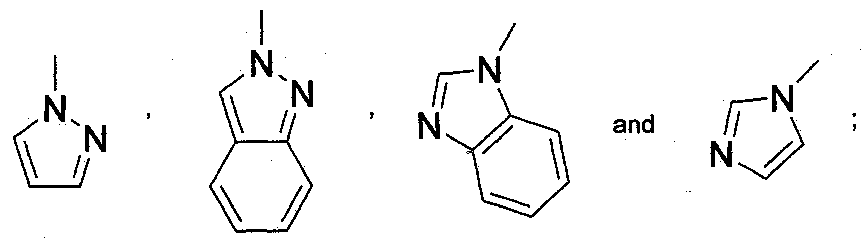 whereby each of these afore mentioned cyclic moieties may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)=C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R¹ and R² together with the bridging nitrogen atom form a nitro group or a moiety selected from the group consisting of 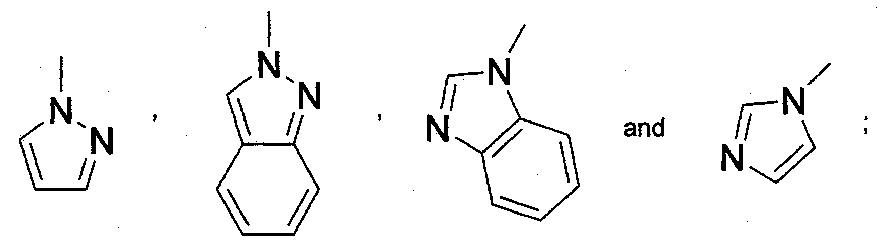 whereby each of these afore mentioned cyclic moieties may be unsubstituted or substituted with 1, 2 or 3 substituent(s) independently selected from the group consisting of methyl, ethyl, n-propyl, iso-propyl, n-butyl, tert-butyl, sec-butyl, iso-butyl, F, Cl, Br, I, -CN and -CF₃,
more preferably R¹ and R² together with the bridging nitrogen atom form a nitro group or a moiety selected from the group consisting of 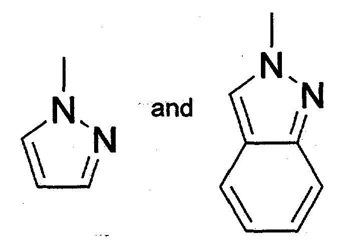 whereby each of these afore mentioned cyclic moieties may be unsubstituted or substituted with 1, 2 or 3 substituent(s) independently selected from the group consisting of methyl, ethyl, n-propyl, iso-propyl, n-butyl, tert-butyl, sec-butyl, iso-butyl, F, Cl, Br, I, -CN and -CF₃,
and X and R³ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R³ represents
a linear or branched C₁₋₁₀ alkyl radical that may be unsubstituted or substituted with 1, 2 or 3 substituent(s) independently selected from the group consisting of -O-C₁₋₅₋alkyl, -S-C₁₋₅-alkyl, F, Cl, Br, I, -CF₃, -OCF₃, -SCF₃, -OH and -SH;
preferably R³ represents an alkyl radical selected from the group consisting of methyl, ethyl, n-propyl, iso-propyl, n-butyl, sec-butyl, iso-butyl and tert-butyl;
more preferably R³ represents a methyl or ethyl radical
and X, R¹, R² and R⁴ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁴ represents
a linear or branched C₁₋₁₀ alkyl radical that may be unsubstituted or substituted with 1, 2 or 3 substituent(s) independently selected from the group consisting of -O-C₁₋₅₋alkyl, -S-C₁₋₅-alkyl, F, Cl, Br, I, -CF₃, -OCF₃, -SCF₃, -OH and -SH;
preferably R⁴ represents an alkyl radical selected from the group consisting of methyl, ethyl, n-propyl, iso-propyl, n-butyl, sec-butyl, iso-butyl and tert-butyl;
more preferably R⁴ represents a methyl or ethyl radical,
and X, R¹ to R³ and R⁵ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁵ represents
a C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁵ represents
an alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
more preferably R⁵ represents
an alkyl radical selected from the group consisting of methyl, ethyl, n-propyl, iso-propyl, n-butyl, sec-butyl, iso-butyl and tert-butyl;
or an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, -O=CH₂-CH₂-CH₃, -0-CH(CH₃)₂, -O-C(CH₃)₃, F, Cl, Br, -CN, -CF₃, -OCF₃, -OH and SH,
and X, R¹ to R⁴ and R⁶ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁶ represents
a hydrogen atom or
an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁶ represents
a hydrogen atom or
an aryl radical selected from the group consisting of phenyl and naphthyl, whereby said aryl radical may be bonded via a -(CH₂)-group and/or may be unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, F, Cl, Br,-CF₃, -OCF₃, -OH and -SH.
more preferably R⁶ represents a hydrogen atom, or
a phenyl radical, whereby said phenyl radical may be bonded via a -(CH₂)-group and/or may be unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, F, Cl, Br,-CF₃, OCF₃, -OH and -SH,
and X, R¹ to R⁵ and R⁷ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁷ represents
a linear or branched C₁₋₁₀ alkyl radical that may be unsubstituted or substituted with 1, 2 or 3 substituent(s) independently selected from the group consisting of -O-C₁₋₅₋alkyl, -S-C₁₋₅-alkyl, F, Cl, Br, I, -CF₃, -OCF₃, -SCF₃, -OH and -SH;
- preferably R⁷ represents an alkyl radical selected from the group consisting of methyl, ethyl, n-propyl, iso-propyl, n-butyl, sec-butyl, iso-butyl and tert-butyl;
more preferably R⁷ represents a methyl or ethyl radical,
and X, R¹ to R⁶ and R⁸ to R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁸ represents
a C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2} or ₃-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁸ represents
an alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2} or ₃-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
more preferably R⁸ represents
an alkyl radical selected from the group consisting of methyl, ethyl, n-propyl, iso-propyl, n-butyl, sec-butyl, iso-butyl and tert-butyl; or
an aryl radical selected from the group consisting of phenyl and naphthyl, whereby said aryl radical may be bonded via a -(CH₂)-, or -(CH₂)-(CH₂)-group and/or may unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH,
and X, R¹ to R⁷, R⁹ and R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R⁹ represents
a C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH;
or an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁹ represents
an alkyl radical selected from the group consisting of methyl, ethyl, n-propyl, iso-propyl, n-butyl, sec-butyl, iso-butyl and tert-butyl; or
an aryl radical selected from the group consisting of phenyl and naphthyl, whereby said aryl radical may be bonded via a -(CH₂)_{1, 2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃,-OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
more preferably R⁹ represents
an alkyl radical selected from the group consisting of methyl, ethyl, n-propyl, iso-propyl, n-butyl, sec-butyl, iso-butyl and tert-butyl;
or an aryl radical selected from the group consisting of phenyl and naphthyl, whereby said aryl radical may be bonded via a -(CH₂)-, -(CH₂)-(CH₂)- or -(CH₂)-(CH₂)-(CH₂)-group and/or may unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, -O-CH₂-CH₂-CH₃, -0-CH(CH₃)₂, -O-C(CH₃)₃, F, Cl, Br, -CN, -CF₃, -OCF₃, -OH and -SH,
and X, R¹ to R⁸ and R¹⁰ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Preferred are also substituted phenyl-piperazine compounds of general formula I given above, wherein R¹⁰ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, - SCF₃, -OH and -SH;
preferably R¹⁰ represents an alkyl radical selected from the group consisting of methyl, ethyl, n-propyl, iso-propyl, n-butyl, sec-butyl, iso-butyl and tert-butyl;
more preferably R¹⁰ represents a methyl or ethyl radical,
and X and R¹ to R⁹ have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Particularly preferred are substituted phenyl-piperazine compounds of general formula I given above, wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thia (=S), C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, -(CH₂)-(CH₂)-, -(CH₂)-(CH₂)-(CH₂)-, -O-(CH₂)-(CH₂)- or-(CH₂)-(CH₂)-O-group and/or may be unsubstituted or substituted with 1, 2 or 3 substituent(s) independently selected from the group consisting of C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), -N(C₁₋₅₋alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R² represents a hydrogen atom or a -C(=O)-R¹⁰ moiety;
R¹ and R² together with the bridging nitrogen atom form a nitro group; or an optionally at least mono-substituted moiety selected from the group-consisting of 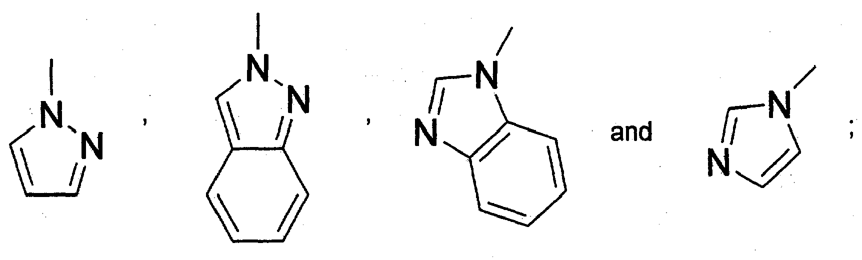 whereby each of these afore mentioned cyclic moieties may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, - C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R³ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and - SH;
R⁴ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and - SH;
R⁵ represents
a C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁶ represents
a hydrogen atom or
an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁷ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and - SH;
R⁸ represents
a C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C-(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁹ represents
a C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, -OH and SH;
or an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R¹⁰ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and - SH,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

Also particularly preferred are substituted phenyl-piperazine compounds of general formula I given above, wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3-} group and/or may be unsubstituted or substituted with 1, 2 or 3 substituent(s) independently selected from the group consisting of oxo (=O), thia (=S), methyl, ethyl, n-propyl, iso-propyl, n-butyl, tert-butyl, sec-butyl, iso-butyl, n-pentyl, -O-CH₃, -O-C₂H₅, -O-CH₂-CH₂-CH₃, -O-CH(CH₃)₂, -O=C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, - C(=O)-O-C₂H₅, -C(=O)-O-CH₃-CH₃=CH₃, -C(=O)-O-CH(CH₃)₂, -C(=O)-O-C(CH₃)₃, - C(=O)-CH₃, -C(=O)-C₂H₅, -C(=O)-CH₃-CH₃-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂₋CH₂-CH₃, -NH-CH(CH₃)₂, -NH-C(CH₃)₃, -N(CH₃)₂, -N(C₂H₅)₂, -NO₂, -CHO, -CF₂H, - CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, -(CH₂)-(CH₂)-, -(CH₂)-(CH₂)-(CH₂)-, -O-(CH₂)-(CH₂)- or - (CH₂)-(CH₂)-O-group and/or may be unsubstituted or substituted with 1, 2 or 3 substituent(s) independently selected from the group consisting of methyl, ethyl, n-propyl, iso-propyl, n-butyl, tert-butyl, sec-butyl, iso-butyl, n-pentyl, -O-CH₃, -O-C₂H₅, - O-CH₂-CH₂-CH₃, -O-CH(CH₃)₂, -O-C(CH₃)₃, -S-CH₃, -S-C₂H₅, -S-CH₂-CH₂-CH₃, -S-CH(CH₃)₂, -S-C(CH₃)₃, -C(=O)-OH, -C(=O)-O-CH₃, -C(=O)-O-C₂H₅, -C(=O)-O-CH₃₋CH₃-CH₃, -C(=O)-O-CH(CH₃)₂, -C(=O)-O-C(CH₃)₃, -C(=O)-CH₃, -C(=O)-C₂H₅, - C(=O)-CH₃-CH₃-CH₃, -C(=O)-CH(CH₃)₂, -C(=O)-C(CH₃)₃, F, Cl, Br, I, -CN, -CF₃, - OCF₃, -SCF₃, -OH, -SH, -NH₂, -NH-CH₃, -NH-C₂H₅, -NH-CH2-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and - S(=O)₂-CH₃,
R² represents a hydrogen atom or a -C(=O)-R¹⁰ moiety;
preferably R¹ and R² together with the bridging nitrogen atom form a nitro group or a moiety selected from the group consisting of 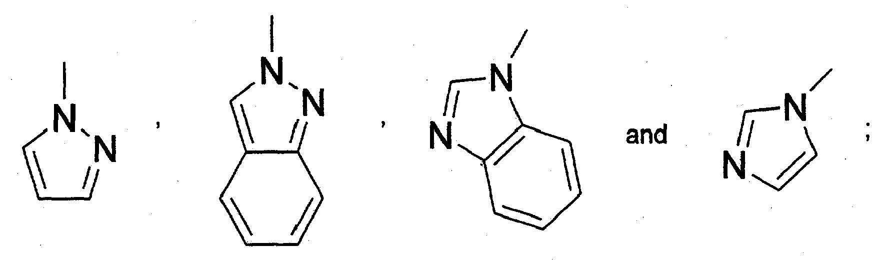 whereby each of these afore mentioned cyclic moieties may be unsubstituted or substituted with 1, 2 or 3 substituent(s) independently selected from the group consisting of methyl, ethyl, n-propyl, iso-propyl, n-butyl, tert-butyl, sec-butyl, iso-butyl, F, Cl, Br, I, -CN and -CF₃,
R³ represents an alkyl radical selected from the group consisting of methyl, ethyl, n-propyl, iso-propyl, n-butyl, sec-butyl, iso-butyl and tert-butyl;
R⁴ represents an alkyl radical selected from the group consisting of methyl, ethyl, n-propyl, iso-propyl, n-butyl, sec-butyl, iso-butyl and tert-butyl;
R⁵ represents
an alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁶ represents
a hydrogen atom or
an aryl radical selected from the group consisting of phenyl and naphthyl, whereby said aryl radical may be bonded via a -(CH₂)-group and/or may be unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, F, Cl, Br,-CF₃, -OCF₃, -OH and -SH,
R⁷ represents an alkyl radical selected from the group consisting of methyl, ethyl, n-propyl, iso-propyl, n-butyl, sec-butyl, iso-butyl and tert-butyl;
R⁸ represents
an alkyl radical selected from the group consisting of methyl, ethyl, n-propyl, iso-propyl, n-butyl, sec-butyl, iso-butyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a -(CH₂)_{1,2 or 3}-group and/or may be unsubstituted or substituted with 1, 2 or 3 substituent(s) independently selected from the group consisting of C₁₋₅₋alkyl, -O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, -NH(C₁₋₅₋alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁹ represents
an alkyl radical selected from the group consisting of methyl, ethyl, n-propyl, iso-propyl, n-butyl, sec-butyl, iso-butyl and tert-butyl; or
an aryl radical selected from the group consisting of phenyl and naphthyl, whereby said aryl radical may be bonded via a -(CH₂)_{1, 2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, -SCF₃, - OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R¹⁰ represents an alkyl radical selected from the group consisting of methyl, ethyl, n-propyl, iso-propyl, n-butyl, sec-butyl, iso-butyl and tert-butyl,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.
Also particularly preferred are substituted phenyl-piperazine compounds of general formula I given above, wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents
a hydrogen atom; or
an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅; -O-CH₂-CH₂-CH₃, -O-CH(CH₃)₂, -O-C(CH₃)₃; F, Cl, Br, -CN, -CF₃, -OCF₃, -OH and -SH. R² represents a hydrogen atom or a -C(=O)-R¹⁰ moiety;
R¹ and R² together with the bridging nitrogen atom form a nitro group or moiety selected from the group consisting of 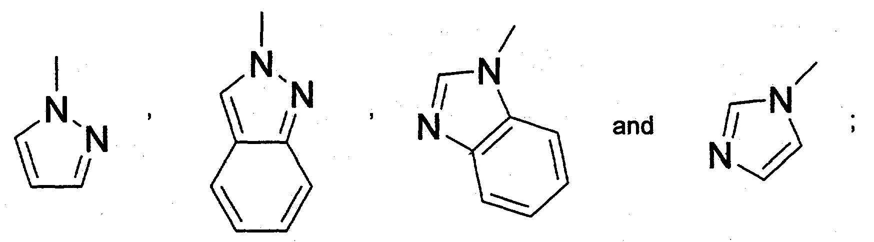 whereby each of these afore mentioned cyclic moieties may be unsubstituted or substituted with 1, 2 or 3 substituent(s) independently selected from the group consisting of methyl, ethyl, n-propyl, iso-propyl, n-butyl, tert-butyl, sec-butyl, iso-butyl, F, Cl, Br, I, -CN and -CF₃,
R³ represents a methyl or ethyl radical;
R⁴ represents a methyl or ethyl radical;
R⁵ represents
an alkyl radical selected from the group consisting of methyl, ethyl, n-propyl, iso-propyl, n-butyl, sec-butyl, iso-butyl and tert-butyl;
or an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)- or -(CH₂)-(CH₂)-(CH₂)- group and/or may unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH;
R⁶ represents a hydrogen atom, or
a phenyl radical, whereby said phenyl radical may be bonded via a -(CH₂)-group and/or may be unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, F, Cl, Br,-CF₃, -OCF₃, -OH and -SH;
R⁷ represents a methyl or ethyl radical;
R⁸ represents
an alkyl radical selected from the group consisting of methyl, ethyl, n-propyl, iso-propyl, n-butyl, sec-butyl, iso-butyl and tert-butyl; or
an aryl radical selected from the group consisting of phenyl and naphthyl, whereby said aryl radical may be bonded via a -(CH₂)-, or -(CH₂)-(CH₂)-group and/or may unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH,
R⁹ represents
an alkyl radical selected from the group consisting of methyl, ethyl, n-propyl, iso-propyl, n-butyl, sec-butyl, iso-butyl and tert-butyl;
or an aryl radical selected from the group consisting of phenyl and naphthyl, whereby said aryl radical may be bonded via a -(CH₂)-, -(CH₂)-(CH₂)- or -(CH₂)-(CH₂)-(CH₂)-group and/or may unsubstituted or substituted with 1, 2 or 3 substituent(s) independently selected from the group consisting of methyl, ethyl, n-propyl, iso-propyl, n-butyl, tert-butyl, sec-butyl, iso-butyl, -O-CH₃, -O-C₂H₅, -O-CH₂-CH₂-CH₃, -0-CH(CH₃)₂, -O-C(CH₃)₃, F, Cl, Br, -CN, -CF₃, -OCF₃, -OH and -SH;
R¹⁰ represents a methyl or ethyl radical,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

More particularly preferred are substituted phenyl-piperazine compounds of general formula I given above, wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents
a hydrogen atom; or
an unsubstituted aryl or heteroaryl radical selected from the group consisting of phenyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or - (CH₂)-(CH₂)-O-group,
R² represents a hydrogen atom or a -C(=O)-R¹⁰ moiety;
or
R¹ and R² together with the bridging nitrogen atom form a nitro group or a cyclic moiety of the following formula: 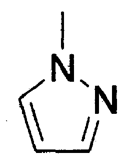 whereby said cyclic moiety may be substituted with 1, 2 or 3 substituent(s) independently selected from the group consisting of methyl, ethyl, n-propyl, iso-propyl, n-butyl, iso-butyl, sec-butyl and tert-butyl;
R³ represents a methyl or ethyl radical;
R⁴ represents a methyl or ethyl radical;
R⁵ represents a methyl radical, an ethyl radical; or an unsubstituted phenyl radical, which may be bonded via a -(CH₂)-group;
R⁶ represents a hydrogen atom or an unsubstituted benzyl radical;
R⁷ represents a methyl or ethyl radical;
R⁸ represents a methyl or ethyl radical; or an unsubstituted phenyl radical;
R⁹ represents an unsubstituted benzyl radical;
and
R¹⁰ represents a methyl or ethyl radical,
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof.

In another aspect the present invention relates to a process for the preparation of a substituted phenyl-piperazine compound of general formula I given above, wherein at least one substituted benzene compound of general formula II, 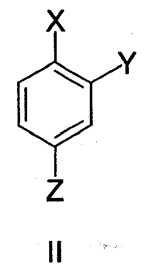 wherein X represents -CN, -C(=O)-OR⁴, -O-R⁵ or -NO₂, R⁴ and R⁵ have any of the above given meanings, Y represents a chlorine atom, and Z represents a bromine or iodine atom; is reacted with at least one piperazine compound of general formula III, 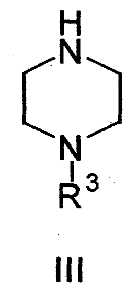 wherein R³ has any of the above given meanings, in a suitable reaction medium, preferably in at least one organic solvent, more preferably in at least one organic solvent selected from the group consisting of tetrahydrofuran, toluene or dioxane, preferably in the presence of at least one catalyst, more preferably in the presence of at least a palladium source, even more preferably in the presence of at least one palladium source selected from the group consisting of Pd(OAc)₂, wherein OAc is acetate, and PdCl₂(dppf), wherein dppf is 1,1-bis(diphenylphosphino)-ferracene, and/or at least one auxiliary agent, preferably at least one auxiliary agent selected from the group consisting of 1,1-bis(diphenylphosphino)-ferrocene and 2,2'-bis(diphenylphosphino)-1'1-binaphthyl (BINAP), optionally in form of its enantiomers or a racemate, and/or at least one base, preferably at least one base selected from the group consisting of sodium *tert*-pentoxide and Cs₂CO₃ to yield a compound of general formula IV, 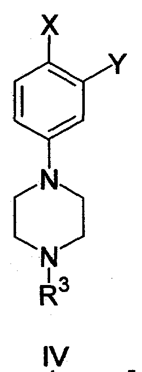 wherein X represents -CN, -C(=O)-OR⁴, -O-R⁵ or -NO₂, R³, R⁴ and R⁵ have any of the above given meanings and Y represents a chlorine atom; which is optionally purified and/or isolated, and the compound of general formula IV is reacted with at least one compound of general formula V, 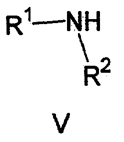 wherein R¹ and R² have any of the above given meanings or one of them represents a protecting group, preferably a -C(=O)-O-C(CH₃)₃ group, in a suitable reaction medium, preferably in at least one organic solvent, more preferably in at least one organic solvent selected from the group consisting of toluene, dioxane and dimethoxyethane, preferably in the presence of at least one catalyst, more preferably in the presence of at least a palladium source, even more preferably in the presence of at least a palladium source selected from the group consisting of Pd(OAc)₂, wherein OAc is acetate, and Pd₂dba₃, wherein dba is dibenzylidene acetone, and/or at least one auxiliary agent, preferably at least one auxiliary agent selected from the group consisiting of (biph)P(*t*Bu)₂, wherein biph is biphenyl and *t*Bu is *tert*-butyl, and 4,5-bis(diphenylphosphino)-9,9-dimethylxanthene (xantphos), and and/or at least one base, preferably at least one base selected from the group consisting of K₃PO₄, Cs₂CO₃ and sodium *tert*-pentoxide to yield a compound of general formula VI, 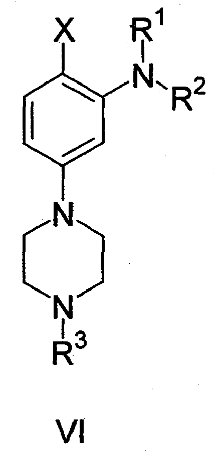 wherein X represents -CN, -C(=O)-OR⁴, -O-R⁵ or -NO₂, R¹, R² have any of the above given meanings or one of them represents a protecting group, preferably - C(=O)-O-C(CH₃)₃ and R³, R⁴ and R⁵ have any of the above given meanings, said compound of general formula VI is being optionally purified and/or isolated,
or at least one substituted benzene compound of general formula IIa, 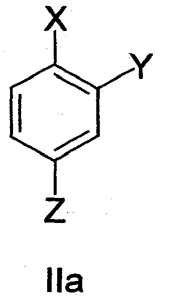 wherein X represents -CN, -C(=O)-OR⁴, -O-R⁵ or -NO₂, R⁴ and R⁵ have any of the above given meanings, Z represents a chlorine atom, Y represents a bromine or iodine atom, is reacted with at least one compound of general formula V, 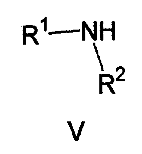 wherein R¹ and R² have any of the above given meanings or one of them represents a protecting group, preferably a -C(=O)-O-C(CH₃)₃ group in a suitable reaction medium, preferably in at least one organic solvent, more preferably in at least one organic solvent selected from the group consisting of toluene, dimethoxyethane and dioxane, preferably in the presence of at least one catalyst, more preferably in the presence of at least a palladium and/or copper source, even more preferably in the presence of at least a palladium and/or copper source selected from the group consisting of Pd(OAc)₂, wherein OAc is acetate, Pd₂dba₃, wherein dba is dibenzylidene acetone, and copper(I)iodide, and/or at least one auxiliary agent, preferably at least an auxiliary agent selected from the group consisting of 4,5-bis(diphenylphosphino)-9,9-dimethylxanthene (Xantphos), 1,1-bis(diphenylphosphino-ferrocene and P(*t*Bu)₃ wherein *t*Bu is tert-Butyl, and/or at least one base, preferably at least one base selected from the group-consisting of K₃PO₄, Cs₂CO₃ and trans-1,2-diamino-methylcyclohexane to yield a compound of general formula VII, 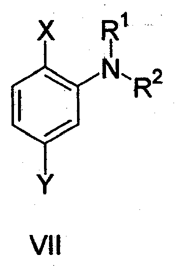 wherein X represents -CN, -C(=O)-OR⁴, -O-R⁵ or -NO₂, R¹ and R² have any of the above given meanings or one of them represents a protecting group, preferably a - C(=O)-O-C(CH₃)₃ group, R⁴ and R⁵ have any of the above given meanings, and Y represents a chlorine atom; said compound of general formula being optionally purified and/or isolated, and the compound of general formula VII is reacted with at least one compound of general formula III, 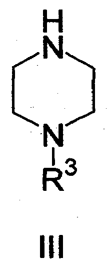 wherein R³ has any of the above given meanings, in a suitable reaction medium, preferably in at least one organic solvent, more preferably in at least one organic solvent selected from the group consisting of tetrahydrofuran, toluene or dioxane, preferably in the presence of at least one catalyst, more preferably in the presence of at least a palladium source, even more preferably in the presence of at least one palladium source selected from the group consisting of Pd(OAc)₂, wherein OAc is acetate, and PdCl₂(dppf), wherein dppf is 1,1-bis(diphenylphosphino)-ferrocene, and/or at least one auxiliary agent, preferably at least one auxiliary agent selected from the group consisting of 1,1-bis(diphenylphosphino)-ferrocene and 2,2'-bis(diphenylphosphino)-1'1-binaphthyl (BINAP), optionally in form of its enantiomers or a racemate, and/or at least one base, preferably at least one base selected from the group consisting of sodium *tert*-pentoxide and Cs₂CO₃, to yield a compound of general formula VI, 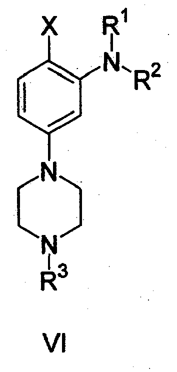 wherein X represents -CN, -C(=O)-OR⁴, -O-R⁵ or -NO₂, R¹ and R² have any of the above given meanings or one of them represents a protecting group, preferably a - C(=O)-O-C(CH₃)₃ group, and R³, R⁴ and R⁵ have any of the above given meanings, and said compound of general formula VI is optionally purified and/or isolated,
or
at least one substituted benzene compound of general formula VIII, 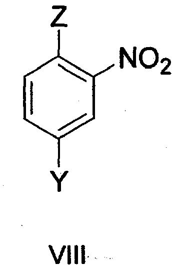 wherein Z represents bromine or iodine and Y represents chlorine, is reacted with at least one compound of general formula IX, 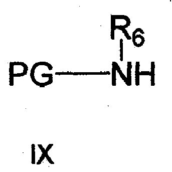 wherein R⁶ has any of the above given meanings and PG represents a protecting group, preferably a-C(=O)-O-C(CH₃)₃ group, in a suitable reaction medium, preferably in at least one organic solvent, more preferably in at least one organic solvent selected from the group consisting of toluene and dioxane, preferably in the presence of at least one catalyst, more preferably in the presence of at least a palladium and/or copper source, even more preferably in the presence of at least a palladium and/or copper source selected from the group consisting of Pd(OAc)₂ wherein OAc is acetate, Pd₂dba₃ wherein dba is dibenzylidene acetone and copper(I)iodide, and/or at least one auxiliary agent, preferably at least an auxiliary agent selected from the group consisting of 4,5-bis(diphenylphosphino)-9,9-dimethylxanthene (Xantphos), 1,1-bis(diphenylphosphino-ferrocene and P(*t*Bu)₃ wherein *t*Bu is tert-Butyl, and/or at least one base, preferably at least one base selected from the group consisting of K₃PO₄, Cs₂CO₃ and trans-1,2-diamino-methylcyclohexane to yield a compound of general formula XI, 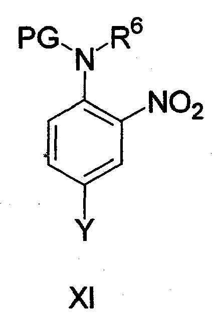 wherein R⁶ has any of the above given meanings, PG represents a protecting group, preferably a-C(=O)-O-C(CH₃)₃ group and Y represents chlorine; which is optionally purified and/or isolated, and the compound of general formula XI reacted with at least one compound of general formula III, 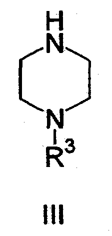 wherein R³ has any of the above given meanings, in a suitable reaction medium, preferably in at least one organic solvent, more preferably in at least one organic solvent selected from the group consisting of tetrahydrofuran, toluene or dioxane, preferably in the presence of at least one catalyst, more preferably in the presence of at least a palladium source, even more preferably in the presence of at least one palladium source selected from the group consisting of Pd(OAc)₂, wherein OAc is acetate, and PdCl₂(dppf), wherein dppf is 1,1-bis(diphenylphosphino)-ferrocene, and/or at least one auxiliary agent, preferably at least one auxiliary agent selected from the group consisting of 1,1-bis(diphenylphosphino)-ferrocene and 2,2'-bis(diphenylphosphino)-1'1-binaphthyl (BINAP), optionally in form of its enantiomers or a racemate, and/or at least one base, preferably at least one base selected from the group consisting of sodium *tert*-pentoxide and Cs₂CO₃, to yield a compound of general formula XII, 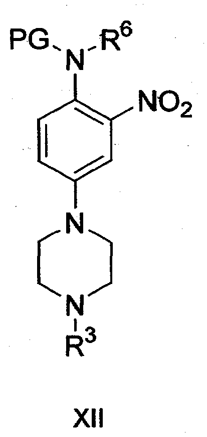 wherein R³ and R⁶ have any of the above given meanings and PG represents a protecting group, preferably a-C(=O)-O-C(CH₃)₃ group, which is optionally purified and/or isolated, and the compound of general formula XII is reacted with at least one acid in a suitable reaction medium to yield a compound of general formula XIII, 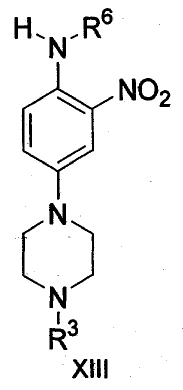 wherein R³ and R⁶ have any of the above given meanings, which is optionally purified and/or isolated, and the compound of general formula is reacted with hydrogen in the presence of at least one catalyst, preferably in the presence of at least one palladium source, more preferably in the presence of palladium on charcoal, in a suitable reaction medium, preferably in at least one organic solvent, more preferably in an organic solvent selected from the group consisting of dioxane, tetrahydrofuran and diethyl ether, to yield a compound of general formula XIV, 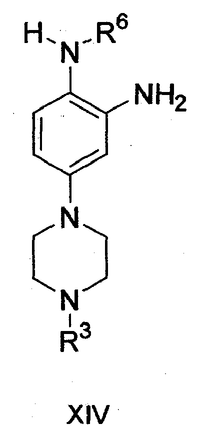 wherein R³ and R⁶ have any of the above given meanings, which is optionally purified and/or isolated, and the compound of general formula XIV is reacted with at least one compound of general formula R⁷-C(=O)-O-C(=O)-R⁷, wherein R⁷ any of the above given meanings, and/or at least one compound of general formula R¹⁰-C(=O)-O-C(=O)-R¹⁰, wherein R¹⁰ has any of the above given meanings, optionally in the presence of at least one base, preferably in the presence of at least one organic base, more preferably in the presence of at least an organic base selected from the group consisting of pyridine, triethylamine and diisopropylethylamine, in a suitable reaction medium, preferably in at least one organic solvent, more preferably in at least one organic solvent selected from the group consisting of dioxane, tetrahydrofuran and diethyl ether, to yield a compound of general formula I, wherein X represents -NR⁶-C(=O)R⁷, R¹ represents a hydrogen atom, R² represents a hydrogen atom or a -C(=O)-R¹⁰-moiety and R³, R⁶, R⁷ and R¹⁰ have any of the above given meanings, which is optionally purified and/or isolated,
and/or at least one compound compound of general formula VI, wherein X represents -CN, -C(=O)-OR⁴ or -O-R⁵, R¹ and R² have any of the above given meanings or one of them represents a protecting group, preferably a -C(=O)-O-C(CH₃)₃-group, R³, R⁴ and R⁵ have any of the above given meanings, is reacted with at least one acid, preferably at least one acid selected from the group consisting of sulfuric acid, hydrochloric acid and acetic acid, in a suitable reaction medium, preferably in at least one organic solvent, more preferably in at least one organic solvent selected from the group consisting of dioxane and tetrahydrofuran, to yield a compound of general formula I, wherein X represents -CN, -C(=O)-OR⁴ or -O=R⁵, R¹ and R³ to R⁵ have any of the above given meanings and R² represents hydrogen, which is optionally purified and/or isolated,
and optionally at least one compound of general formula I, wherein X represents - CN, -C(=O)-OR⁴ or -O-R⁵, R¹ and R³ to R⁵ have any of the above given meanings and R² represents hydrogen, is reacted with hydrogen in the presence of at least one catalyst, preferably in the presence of at least one palladium source, more preferably in the presence of palladium on charcoal, in a suitable reaction medium, preferably in at least one organic solvent, more preferably in an organic solvent selected from the group consisting of dioxane, tetrahydrofuran and diethyl ether, to yield a compound of general formula I, wherein X represents -CN, -C(=O)-OR⁴ or -O-R⁵, R³ to R⁵ have any of the above given meanings and R¹ and R² each represent hydrogen,
**and/or**
at least one compound of general formula VI, wherein X represents -C(=O)-OR⁴, R¹ and R² have any of the above given meanings or one of them represents a protecting group, preferably a -C(=O)-O-C(CH₃)₃-group, R³ and R⁴ have any of the above given meanings, is reacted with at least one base, preferably at least one metal hydroxide, more preferably at least one metal hydroxide selected from the group consisting of lithium hydroxide and potassium hydroxide, in a suitable reaction medium, preferably in a mixture of at least one organic solvent and water, more preferably in a mixture of at least one organic solvent selected from the group consisting of dioxane, ethanol and methanol and water, to yield a compound of general formula XV, wherein X represents -C(=O)-OH, R¹ and R² have any of the above given meanings or one of them represents a protecting group, preferably a -C(=O)-O-C(CH₃)₃-group, R³ has any of the above given meanings, which is optionally purified and/or isolated and at least one compound of general formula XV is reacted with at least one acid, preferably at least one acid selected from the group consisting of sulfuric acid, hydrochloric acid and acetic acid, in a suitable reaction medium, preferably in at least one organic solvent, more preferably in at least one organic solvent selected from the group consisting of dioxane and tetrahydrofuran, to yield a compound of general formula I, wherein X represents -C(=O)-OH, R¹ and R³ have any of the above given meanings and R² represents hydrogen, which is optionally purified and/or isolated,
and/or
at least one compound of general formula VI, wherein X represents -NO₂, R¹ and R² have any of the above given meanings or one of them represents a protecting group, preferably a -C(=O)-O-C(CH₃)₃-group and R³ has any of the above given meanings, is reacted with hydrogen in the presence of at least one catalyst, preferably in the presence of at least one palladium source, more preferably in the presence of palladium on charcoal, in a suitable reaction medium, preferably in at least one organic solvent, more preferably in an organic solvent selected from the group consisting of dioxane, tetrahydrofuran and diethyl ether, to yield a compound of general formula XVI, wherein X represents -NH₂, R¹ and R² have any of the above given meanings or one of them represents a protecting group, preferably a -C(=O)-O-C(CH₃)₃-group, and R³ has any of the above given meanings, which is optionally purified and/or isolated, and at least one compound of general formula XVI, is reacted with at least one acid, preferably at least one acid selected from the group consisting of sulfuric acid, hydrochloric acid and acetic acid, in a suitable reaction medium, preferably in at least one organic solvent, more preferably in at least one organic solvent selected from the group consisting of dioxane and tetrahyrofuran, to yield a compound of general formula I, wherein X represents -NH₂, R¹ and R³ have any of the above given meanings and R² represents hydrogen, which is optionally purified and/or isolated,
and optionally at least one compound of general formula I, wherein X represents - NH₂, R¹ and R³ have any of the above given meanings and R² represents hydrogen, is reacted with at least one compound of general formula R⁷-C(=O)-O-C(=O)-R⁷ and/or at least one compound of general formula R¹⁰-C(=O)-O-C(=O)-R¹⁰, wherein R⁷ and R¹⁰ have any of the above given meanings optionally in the presence of at least one base, preferably in the presence of at least one organic base, more preferably in the presence of at least an organic base selected from the group consisting of pyridine, triethylamine and diisopropylethylamine, in a suitable reaction medium, preferably in at least one organic solvent, more preferably in at least one organic solvent selected from the group consisting of dioxane, tetrahydrofuran and diethyl ether, to yield a compound of general formula I, wherein X represents -NH-C(=O)-R⁷ and R¹ to R³ have any of the above given meanings, which is optionally purified and/or isolated,
and/or optionally at least one compound of general formula I, wherein X represents - NH₂ and R¹ and R³ have any of the above given meanings and R² represents hydrogen, is reacted with at least one compound of general formula R⁸-S(=O)-W, wherein R⁸ has any of the above given meanings and W represents a halogen atom, preferably a chlorine atom, optionally in the presence of at least one base, preferably in the presence of at least one organic base, more preferably in the presence of an organic base selected from the group consisting of pyridine, triethylamine and diisopropylethylamine, in a suitable reaction medium, preferably in at least one organic solvent, more preferably in at least one organic solvent selected from the group consisting of dioxane, tetrahydrofuran and diethyl ether, to yield a compound of general formula I, wherein X represents -NH-S(=O)₂-R⁸ and R¹, R³ and R⁸ have any of the above given meanings and R² represents hydrogen, which is optionally purified and/or isolated.

Suitable reaction media include organic solvents, such as dialkyl ether, preferably diethyl ether and dimethoxyethane, or a cyclic ether, preferably tetrahydrofuran or dioxane; or a halogenated hydrocarbon, preferably dichloromethane or chloroform; an alcohol, preferably methanol or ethanol; an aprotic solvent, preferably acetonitrile, pyridine, toluene or dimethylformamide, or any other suitable reaction medium. Of course, mixtures of at least two classes of solvents or of at least two solvents of one class may also be used.

If the above mentioned reactions are carried out in an oven-dried vial, the catalyst, the auxiliary agent, the base and the compound of general formula II, IIa, IV, VII, VIII or XI are added in each case and the vial is subsequently evacuated and purged with argon. The organic solvent and the compound of general formula III, V and IX are added and the reaction is carried out in a sealed vial at a temperature between 100 °C and 110 °C, preferably at 100 °C in case of tetrahydrofurane or toluene as the organic solvent and at 110 °C in case of dimethoxyethane and dioxane as the organic solvent.

Suitable reaction conditions for carrying out the reaction between compounds of general formula II, IIa, IV, VII, VIII or XI and compounds of general formula III, V and IX are described in the references of J.F. Hartwig et al., J. Am. Chem. Soc. 1996, 118, 7217-7218; S.L. Buchwald et al., J. Org. Chem. 2000, 65, 1144-1157; S.L. Buchwald et al., J. Am. Chem. Soc. 2002, 124, 6043-6048; S. L. Buchwald et al. J. Am. Chem. Soc. 2002, 124, 7241-7424 and S.L. Buchwald et al., J. Am. Chem. Soc. 2002, 124, 11684-11688. The respective part of the description is hereby incorporated by reference and forms part of the present disclosure.

The compounds of general formula I, IV, VI, VII, XI, XII, XIII, XIV, XV and XVI may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

Preferably, the compounds of general formula I, IV, VI, VII, XI, XII, XIII, XIV, XV and XVI may be obtained by filtration of the reaction mixture and subsequent separation of the reaction mixture on a TLC plate. Alternatively, the compounds of general formula I, IV, VI, VII, XI, XII, XIII, XIV, XV and XVI may be isolated by addition of water and methanol to the reaction mixture, evaporating the reaction mixture and purifying the residue by preparative HPLC.

The compounds of general formula II, IIa, VIII and IX are commercially available or may be prepared according to methods well known in the art, for example, analogous to the methods described in the bibliography of A. McKillop et al., Tetrahedron 1987, 43, 1753. The respective part of the literature description cited above is hereby incorporated by reference and forms part of the disclosure.

During some synthetic reactions described above or while preparing the compounds of general formulas II, IIa, VIII and IX the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the substituted phenyl-piperazine compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents.

The substituted phenyl-piperazine compounds of general formula I and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above.

The term "salt" is to be understood as meaning any form of the substituted phenyl-piperazine compounds of general formula I in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the subsituted phenyl-piperazine compounds of general formula I or in each case of corresponding stereoisomers may also be obtained by standard procedures known to those skilled in the art.

The term "solvate" according to this invention is to be understood as meaning any form of the substituted phenyl-piperazine compounds of general formula I in which they have attached to it via non-covalent binding another molecule (most likely a polar solvent) especially including hydrates and alcoholates, e.g. methanolate.

A further aspect of the present invention relates to a medicament comprising at least one substituted phenyl-piperazine compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one physiologically acceptable auxiliary agent.

Said medicament is particularly suitable for 5-HT₆-receptor regulation and therefore for the prophylaxis and/or treatment of a disorder or a disease that is least partially mediated via 5-HT₆-receptors.

Preferably said medicament is suitable for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

More preferably said medicament is suitable for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

More preferably said medicament is suitable for improvement of cognition (cognitive enhancement) or cognitive memory (cognitive memory enhancement).

Most preferably, said medicament is suitable for the prophylaxis and/or treatment of obesity and/or disorders or diseases related thereto.

In another aspect the present invention relates to the use of at least one substituted phenyl-piperazine compound of general formula I given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament suitable for 5-HT₆-receptor regulation, preferably for the prophylaxis and/or treatment of a disorder or a disease that is least partially mediated via 5-HT₆-receptors.

In another aspect the present invention relates to the use of at least one substituted phenyl-piperazine compound of general formula I given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

The use of at least one substituted phenyl-piperazine compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder) is also preferred.

The use of at least one substituted phenyl-piperazine compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the improvement of cognition (cognitive enhancement) and/or for the improvement of cognitive memory (cognitive memory enhancement) is also preferred.

The use of at least one substituted phenyl-piperazine compound of general formula I given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the prophylaxis and/or treatment of drug addiction and/or withdrawal, preferably for the prophylaxis and/or treatment of addiction and/or withdrawal related to one or more of drugs selected from the group consisting of benzodiazepines, natural, semisynthetic or synthetic opioids like cocaine, ethanol and/or nicotine is also preferred.

More preferred is the use of at least one substituted phenyl-piperazirte compound of general formula I as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the maintenance, increase or reduction of body weight, for the prophylaxis and/or treatment of obesity, bulimia, anorexia, cachexia or type II diabetes (non insulin dependent diabetes mellitus), preferably type II diabetes that is caused by obesity.

More preferred is also the use of at least one substituted phenyl-piperazine compound of general formula I as defined above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament for the improvement of cognition (cognitive enhancement) and/or for the improvement of cognitive memory (cognitive memory enhancement).

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may-contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are are known from the prior art, e.g. from the table of contents of"Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective phenyl-piperazine compound is liberated in the intestinal tract. Preferably the enteric coating is soluble at a pH value of 5 to 7.5. Suitable materials and methods for the preparation are are known from the prior art.

Typically, the medicaments according to the present invention may contain 1-60 % by weight of one or more substituted phenyl-piperazine compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000 mg, preferably 1 to 1500 mg, more preferably 1 to 1000 mg of active substance to be administered during one or several intakes per day.

In the following methods for determining the pharmacological activity of the substituted phenyl-piperazine compounds are described.

Pharmacological Methods:

### I) BINDING TO SEROTONIN RECEPTOR 5-HT₆

Cell membranes of HEK-293 cells expressing the 5HT₆-human recombinant receptor were supplied by Receptor Biology. In said membranes the receptor concentration is 2.18 pmol/mg protein and the protein concentration is 9.17 mg/ml. The experimental protocol follows the method of B. L. Roth et al. [B. L. Roth, S. C. Craigo, M. S. Choudhary, A. Uluer, F. J. Monsma, Y. Shen, H. Y. Meltzer, D. R. Sibley: Binding of Typical and Atypical Antipsychotic Agents to 5-Hydroxytryptamine-6 and Hydroxytryptamine-7 Receptors. The Journal of Pharmacology and Experimental Therapeutics, 1994, 268, 1403] with the following slight changes. The respective part of the literature description is hereby incorporated by reference and forms part of the disclosure.

The commercial membrane is diluted (1:40 dilution) with the binding buffer: 50 mM Tris-HCl, 10 mM MgCl₂, 0.5 mM EDTA (pH 7.4). The radioligand used is [³H]-LSD at a concentration of 2.7 nM with a final volume of 200 µl. Incubation is initiated by adding 100 µl of membrane suspension, (≈ 22.9 µg membrane protein), and is prolonged for 60 minutes at a temperature of 37 °C. The incubation is ended by fast filtration in a Brandel Cell Harvester through fiber glass filters made by Schleicher & Schuell GF 3362 pretreated with a solution of polyethylenimine at 0.5 %. The filters are washed three times with three milliliters of buffer Tris-HCl 50 mM pH 7.4. The filters are transferred to flasks and 5 ml of Ecoscint H liquid scintillation cocktail are added to each flask. The flasks are allowed to reach equilibrium for several hours before counting with a Wallac Winspectral 1414 scintillation-counter. Non-specific binding is determined in the presence of 100 µM of serotonin. Tests were made in triplicate. The inhibition constants (Kᵢ, nM) were calculated by non-linear regression analysis using the program EBDA/LIGAND described in Munson and Rodbard, Analytical Biochemistry, 1980, 107, 220, the respective part of which is hereby incorporated by reference and forms part of the disclosure.

### II.) FOOD INTAKE MEASUREMENT (BEHAVIOURAL MODEL):

Male W rats (200-270 g) obtained from Harlan, S.A. are used. The animals are acclimatized to the animal facility for at least 5 days before they are subjected to any treatment. During this period the animals are housed (in groups of five) in translucid cages and provided with food and water ad libitum. At least 24 hours before the treatment starts, the animals are adapted to single-housing conditions.

The acute effect of the substituted phenyl-piperazine compounds according to the present invention in fasted rats is then determined as follows:
The rats were fasted for 23 hours in their single homecages. After this period, the rats are orally or intraperitoneally dosed with a composition comprising a substituted phenyl-piperazine compound or a corresponding composition (vehicle) without said substituted phenyl-piperazine compound. Immediately afterwards, the rat is left with preweighed food and cumulative food intake is measured after 1, 2, 4 and 6 hours.

Said method of measuring food intake is also described in the literature publications of Kask et al., European Journal of Pharmacology 414 (2001), 215-224 and Turnbull et al., Diabetes, Vol. 51, August 2002. The respective parts of the descriptions are hereby incorporated by reference and form part of the disclosure.

The present invention is illustrated below with the aid of examples. These illustrations are given solely by way of example and do not limit the general spirit of the present invention.

### Examples

### Abbreviations

- aq.: aqueous
- biph: biphenyl
- BINAP: 2,2'-bis(diphenylphosphino)-1'1-binaphthyl
- dba: dibenzylidene acetone
- DCM: dichlormethane
- DME: 1,2-dimethoxyethan
- dppf: 1,1-bis(diphenylphosphino)-ferrocene
- EA: ethyl acetate
- eq.: equivalent
- EtOH: ethanol
- h: hours
- HPLC: high performance liquid chromatography
- MeOH: methanol
- OAc: acetate
- OtPent: *tert*-pentoxide
- PE: petroleum ether
- prep.: preparative
- PTFE: polytetrafluorethylene
- rac: racemic
- rt: room temperature
- *t*Bu: tert-butyl
- THF: tetrahydrofuran
- TLC: thin layer chromatography
- xantphos: 4,5-bis(diphenylphosphino)-9,9-dimethylxanthene

### 1a. Preparation of example compounds 2, 5, 9 to 14 and 26

### Step 1 for preparation of example compounds 2, 5, 9-14 and 26

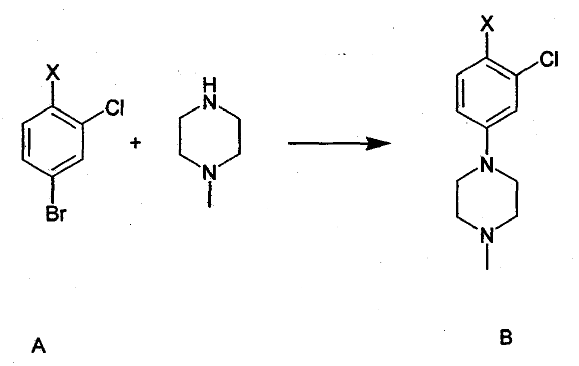

An oven-dried vial was charged subsequently with 0.05 eq. PdCl₂(dppf), 0.15 eq. dppf, 1.0 eq. aryl halide A with X = -O-Benzyl, -O-Methyl or -CN and 1.25 eq. sodium *tert*-pentoxide. The vial was evacuated and purged with argon, and THF and 1-methyl-piperazine (1.1 eq.) were added. THF was added in a quantity to obtain a 0.5 M solution of the aryl halide A. The vial was sealed and heated to 100°C for 3 h. The reaction mixture was taken up in half-saturated brine and extracted three times with ethyl acetate. The volume of the combined organic phases was reduced in vacuo and extracted with 5% hydrochloric acid in H₂O. The acidic aqueous phase was concentrated and the product was isolated by preparative HPLC. In order to remove any ammonium formate derivative formed during preparative HPLC on RP18, the resulting product was taken up in chloroform, extracted twice with saturated aqueous NaHCO₃, the combined aqueous phases were re-extracted with chloroform, and the combined organic phases were dried over MgSO₄ and concentrated in vacuo to obtain the desired product B.

Said process was carried out according to the method described in the reference of J.F. Hartwig et al., J. Am. Chem. Soc. 1996, 118, 7217-7218. The respective part of the description is hereby incorporated by reference and forms part of the present disclosure.

### Step 1 for preparation of example compounds 4, 6 and 7:

An oven-dried vial was charged subsequently with 0.03 eq. Pd(OAc)₂, 0.045 rac-BINAP, 1.4 eq Cs₂CO₃ and 1.0 eq. aryl halide A with X = -C(=O)-OCH₃. The vial was evacuated and purged with argon, and toluene and 1-methyl-piperazine (1.1 eq.) were added. Toluene was added in a quantity to obtain a 0.5 M solution of the aryl halide A. The vial was sealed and heated to 100°C for 19 h.
The reaction mixture was taken up in half-saturated brine and extracted three times with ethyl acetate. The combined organic phases were dried over MgSO₄, concentrated an purified by prep HPLC (RP18). In order to remove any ammonium formate derivative formed during preparative HPLC on RP18, the resulting product was taken up in chloroform, extracted twice with saturated aqueous NaHCO₃, the combined aqueous phases were re-extracted with chloroform, and the combined organic phases were dried over MgSO₄ and concentrated in vacuo to obtain the desired product B.

Said process was carried out according to the method described in the reference of L. Buchwald et al., J. Org. Chem. 2000, 65, 1144-11.57. The respective part of the description is hereby incorporated by reference and forms part of the present disclosure.

### Step 2 for preparation of example compounds 2, 4-7, 9-14 and 26

An oven-dried vial was subsequently charged with 0.1 eq. of a palladium source [Pd], 0.2 eq. (biph)P(*t*Bu)₂, 1.0 eq. arylhalide B with X = -O-Benzyl, -O-Methyl, -CN or - C(=O)-OCH₃ and 1.4 eq. base. The vial was evacuated and purged with argon, and the respective amine HNR¹R² (1.3 eq.) was added. The solvent was added to obtain a concentration of 1.0 M of the arylhalide B. The vial was sealed and heated to either 100°C in case of toluene as the solvent or 110°C in case of DME as the solvent in each case for 22 h.

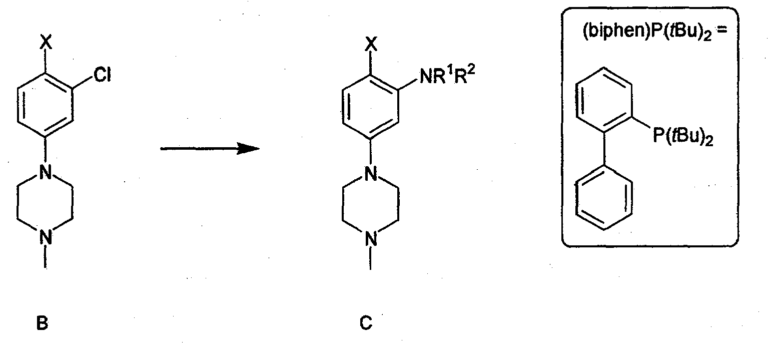

The reaction mixture was transferred into a flask using H₂O and MeOH and the total volume was reduced to less than 10 mL. Purification was achieved by prep. HPLC. In order to remove any ammonium formate derivative formed during preparative HPLC, the resulting product was taken up in chloroform, extracted twice with saturated aq. NaHCO₃, the combined aq. phases were re-extracted with chloroform, and the combined organic phases were dried over MgSO₄. If any additional workup was applied, this is indicated in the table 1.

Said process was carried out according to the method described in the reference of S.L. Buchwald et al., J. Am. Chem. Soc. 2002,124, 6043-6048. The respective part of the description is hereby incorporated by reference and forms part of the present disclosure.

The different reaction conditions are listed in the following table 1.

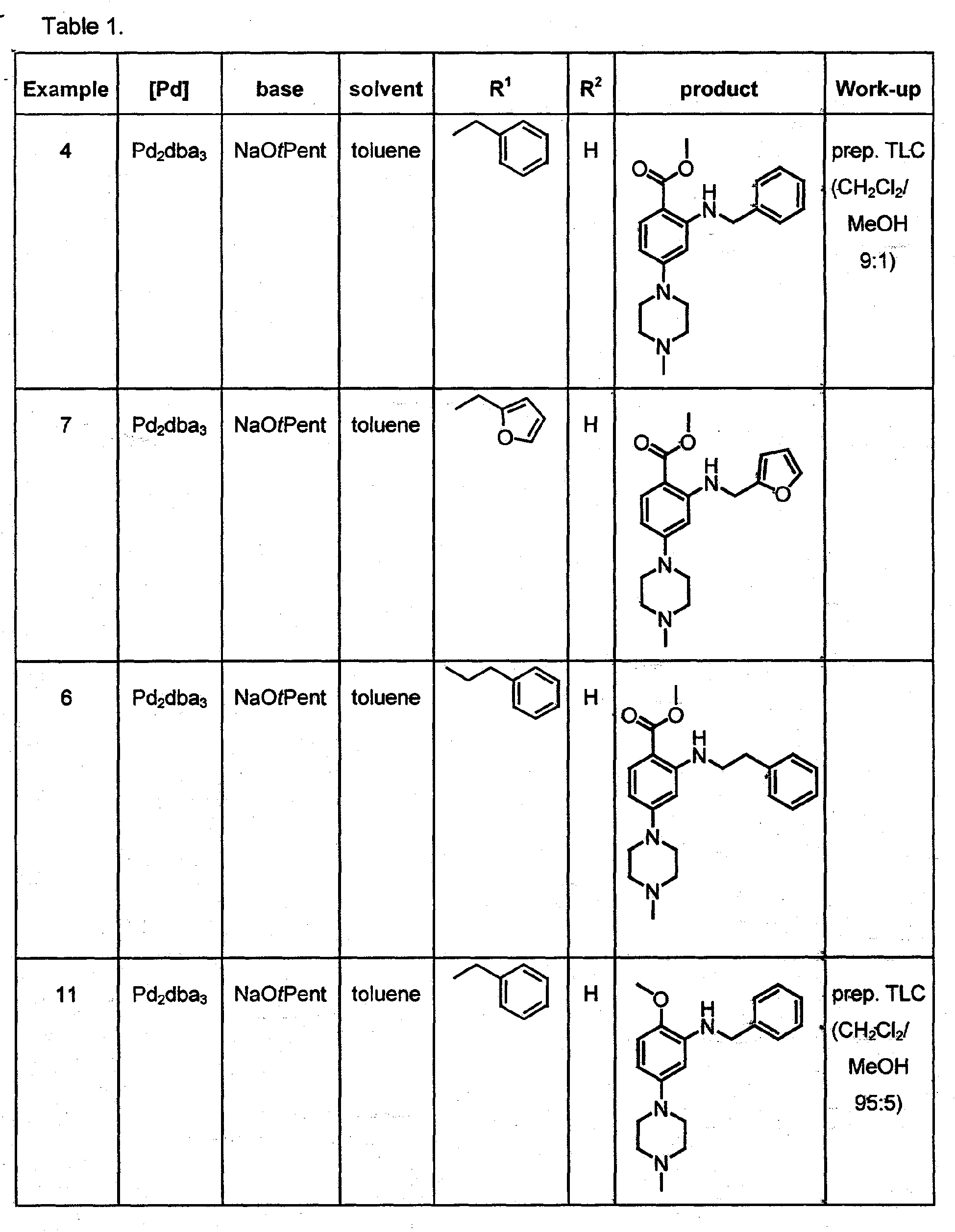

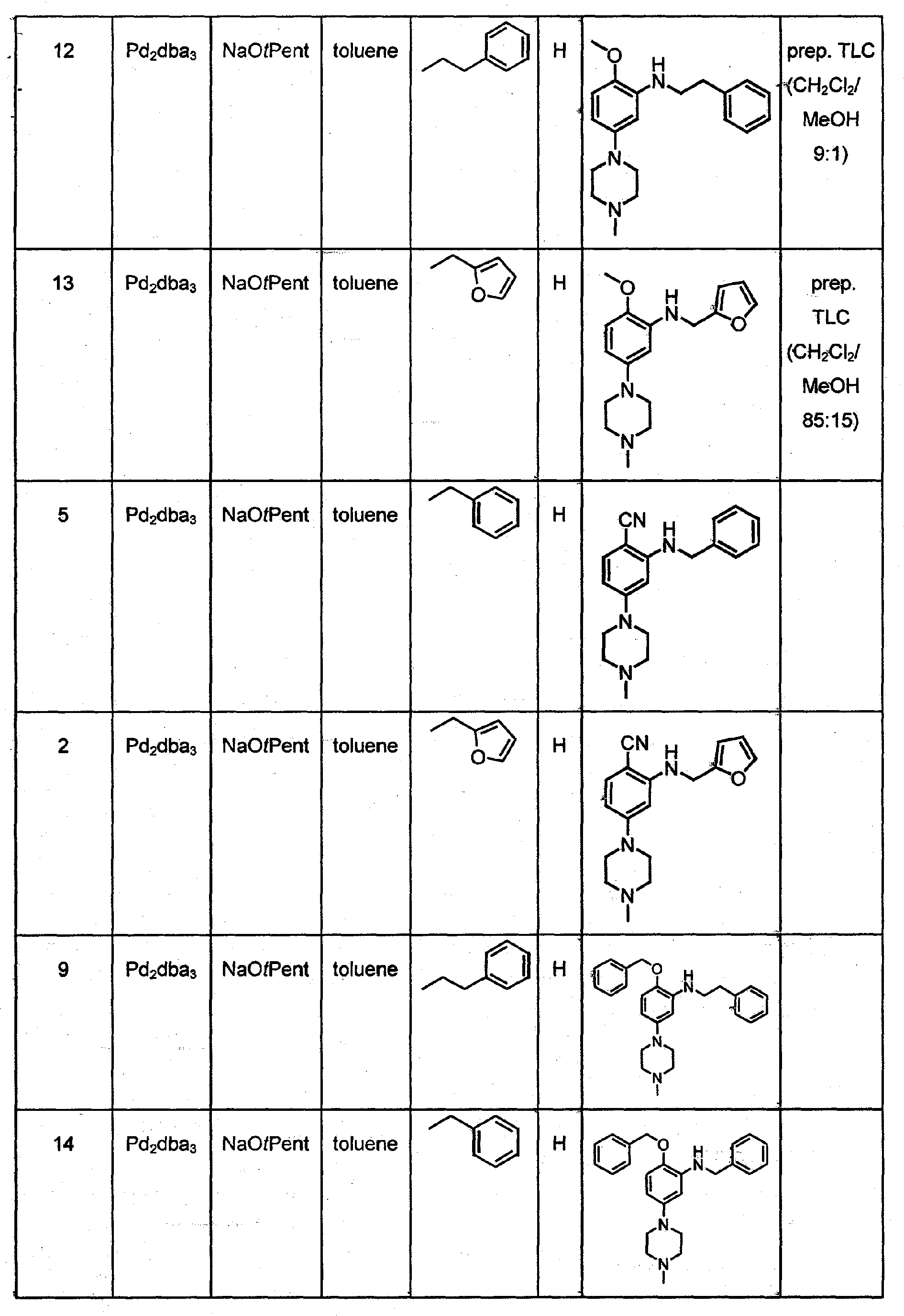

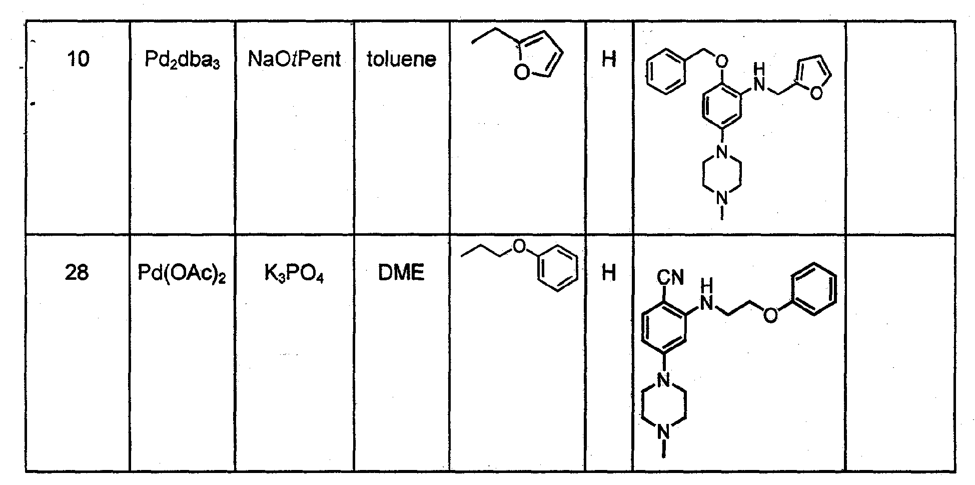

### 1b. Preparation of example compound 26

The example compound 14 was dissolved in THF, 0.1 eq. 5 % palladium on charcoal was added and the mixture was stirred in a hydrogen atmosphere at rt for 16 hours and at 50 °C for 24 hours. The palladium on charcoal was removed by filtration (0.45 µm PTFE-filter). The crude material was pre-purified by prep. TLC (1 mm silica gel, a plate for 0.4 mmol, solvent DCM/Methanol 9:1 VolumeNolume). The product was finally isolated by prep. HPLC (RP 18).

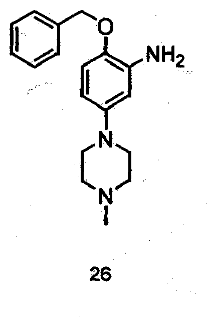

### 2. Preparation of example compounds 1, 3 and 8

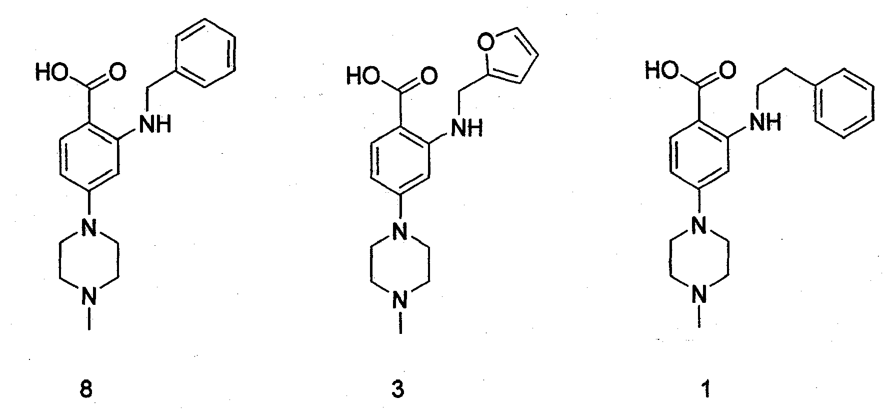

The example compounds 1, 3 or 8 were prepared from the respective methyl benzoate 6, 7 or 4. The methyl benzoate compound (1.0 eq.) was dissolved in a solvent mixture of dioxane and H₂O in a ratio of 50:1 and LiOH•H₂O (5.0 eq.) was added. The resulting mixture was stirred at rt overnight.

Alternatively, the respective methyl benzoate compound 4, 7 or 6 (1.0 eq.) was dissolved in a solvent mixture of EtOH and H₂O in a ratio of 10:1 and potassium hydroxide (5.0 eq.) was added. The mixture was stirred under reflux overnight.

The reaction mixtures were in each case slightly acidified to pH 6 using 5 % aq. hydrochloric acid, the solvent was removed in vacuo and the remaining solid was taken up in 10 mL MeOH and H₂O. The desired product was purified by using prep. HPLC or crystallisation from a small amount of MeOH.

### 3. Preparation of example compounds 15 and 16

### 3a. Preparation of intermediate compound 15-1

The starting material 4-bromo-2-chloro-1-nitro-benzene was obtained by oxidation from 4-bromo-2-chloro-aniline according to the method described in the reference by A. McKillop et al., Tetrahedron 1987, 43, 1753-1758.

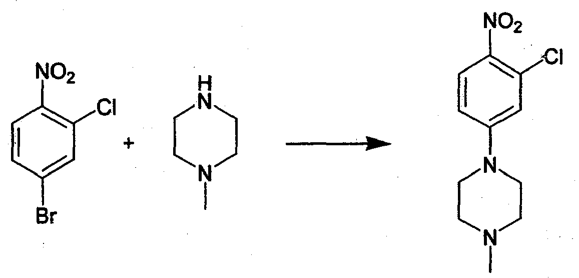

### Step 1:

An oven-dried vial was charged subsequently with 0.05 eq. PdCl₂(dppf), 0.15 eq. dppf, 1.0 eq. 4-bromo-2-chloro-1-nitro-benzene and 1.25 eq. sodium *tert*-pentoxide. The tube was evacuated and purged with argon, and THF and 1-methyl-piperazine (1.1 eq.) were added. THF was added in a quantity to obtain a 0.5 M solution of 4-bromo-2-chloro-1-nitro-benzene. The vial was sealed and heated to 100°C for 3 h. The reaction mixture was taken up in half-saturated brine and extracted three times with ethyl acetate. The volume of the combined organic phases was reduced in vacuo and extracted with 5% hydrochloric acid in H₂O. The acidic aqueous phase was concentrated and the product was isolated by preparative HPLC. In order to remove any ammonium formate derivative formed during preparative HPLC, the resulting product was taken up in chloroform, extracted twice with saturated aqueous NaHCO₃, the combined aqueous phases were re-extracted with chloroform, and the combined organic phases were dried over MgSO₄ and concentrated in vacuo to obtain the desired product.

### Step 2:

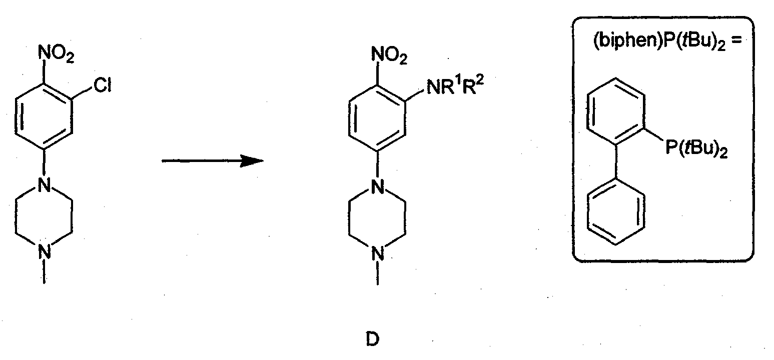

An oven-dried vial was subsequently charged with 0.1 eq. of a palladium source [Pd], 0.2 eq. (biph)P(*t*Bu)₂, 1.0 eq. 1-(3-chloro-4-nitro-phenyl)-4-methyl-piperazine and 1.4 eq. base. The vial was evacuated and purged with argon, and the respective amine HNR¹R² (1.3 eq.) was added. The solvent was added to obtain a concentration of 1.0 M of the arylhalide 1-(3-chloro-4-nitro-phenyl)-4-methyl-piperazine. The vial was sealed and heated to 110°C for 22 h.
The reaction mixture was transferred into a flask using H₂O and MeOH and the total volume was reduced to less than 10 mL. Purification was achieved by prep. HPLC. In order to remove any ammonium formate derivative formed during preparative HPLC, the resulting product was taken up in chloroform, extracted twice with saturated aq. NaHC03, the combined aq. phases were re-extracted with chloroform, and the combined organic phases were dried over MgSO₄.

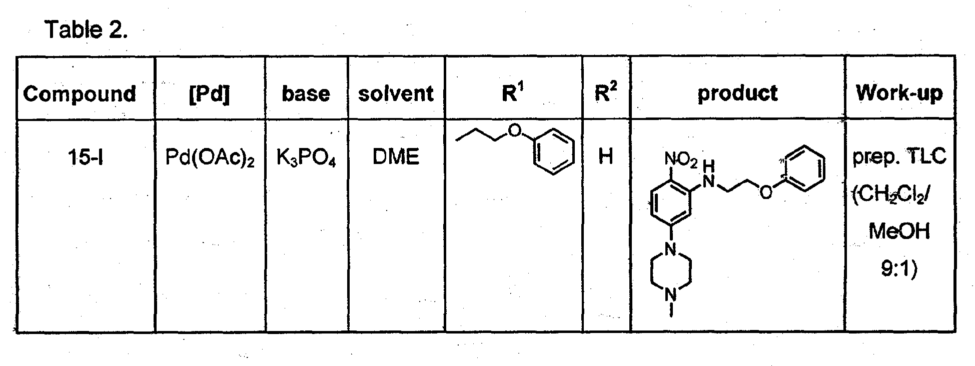

### 3b. Preparation of example compounds 15 and 16

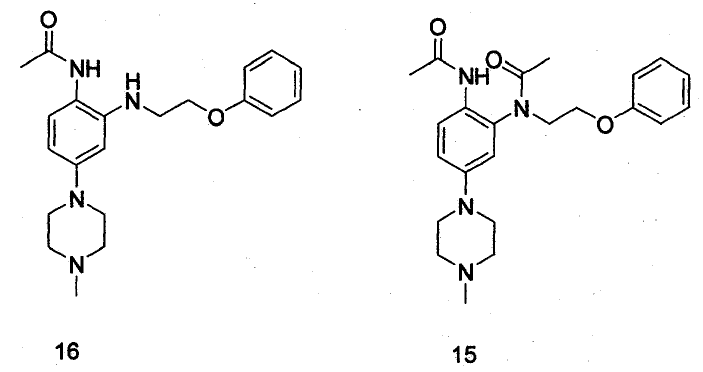

The compound 15-I was dissolved in THF, 0.1 eq. 5 % palladium on charcoal was added and the mixture was stirred in a hydrogen atmosphere at rt for 5 hours. The palladium on charcoal was removed by filtration (filter 0.45 µm PTFE) and the filtrate was directly used in the next reaction.
The filtrate was treated with 1.0 eq. acetanhydride at rt for 5 hours. The reaction mixture was adjusted to pH 7-8 using aq. sat. NaHCO₃ and the product was extracted with DCM. The organic layer was dried over MgSO₄, concentrated and purified by prep. TLC to obtain both 15 and 16.

### 4. Preparation of example compounds 17, 18, 19 to 22 and 24 to 25

The starting material 2-bromo-4-chloro-1-nitro-benzene was obtained by oxidation from 2-bromo-4-chloro-aniline according to the method described in the reference by A. McKillop et al., Tetrahedron 1987, 43,1753-1758.

### 4a. Preparation of compounds 19-I and 20-I

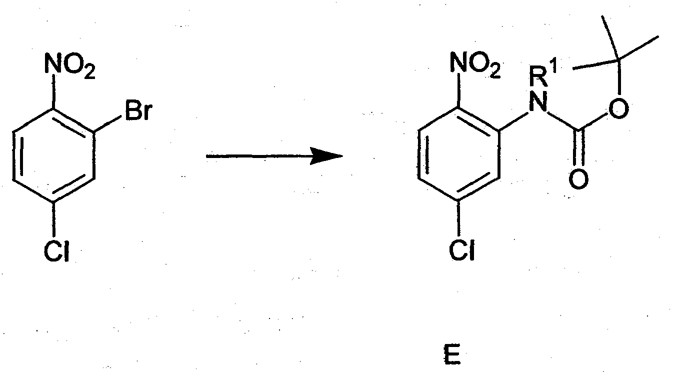

An oven-dried vial was subsequently charged with a palladium source [Pd], xantphos (1.5 eq. to Pd), 1.0 eq. 2-bromo-4-chloro-1-nitro-benzene, 1.1 eq. carbamate HNR¹[C(=O)-O-C(CH₃)₃] and 1.4 eq. base. The tube was evacuated, purged with argon and dioxane was added to obtain a concentration of 1.0 M for 2-bromo-4-chloro-1-nitro-benzene. The vial was sealed and heated to 110°C for 21 h.
The reaction mixture was directly mounted on silica gel and separated using Flash Master II (50 g silica gel cartridge, gradient from 100 % petroleum ether to 50:50 PE/EA) to obtain the desired product.

Said process was carried out according to the method described in the reference of S.L. Buchwald et al., J. Am. Chem. Soc. 2002, 124, 6043-6048. The respective part of the description is hereby incorporated by reference and forms part of the present disclosure.

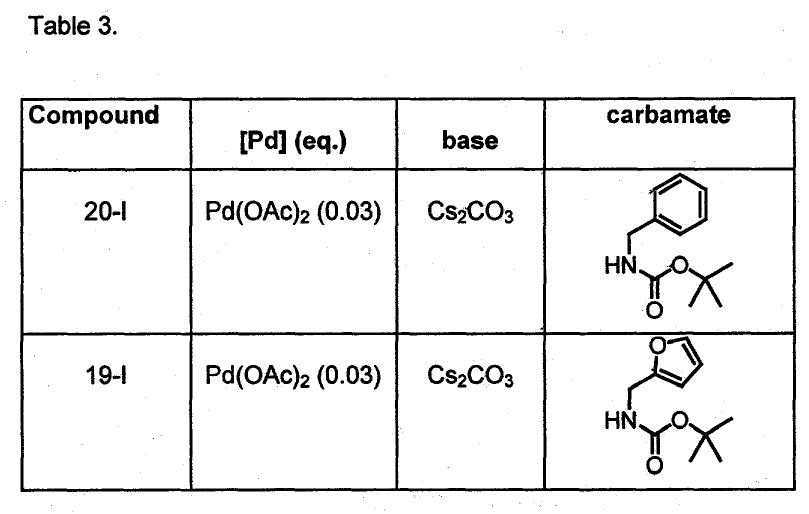

### 4b. Preparation of compound 18-I

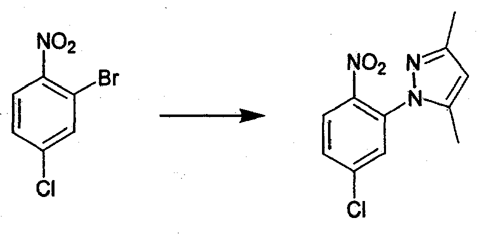

An oven-dried vial was subsequently charged with 0.1 eq. Cul, 1.0 eq. 2-bromo-4-chloro-1-nitro-benzene, 1.2 eq. 3,5-dimethylpyrazole and 2.1 eq. K₃PO₄. The vial was evacuated and purged with argon and 0.3 eq. *trans*-1,2-diaminomethyl-cyclohexane was added. Toluene was added to obtain a concentration of 1.0 M for 2-bromo-4-chloro-1-nitro-benzene . The vial was sealed and heated to 110°C for 21 h.
The mixture was filtered through a pipette stuffed with cotton wool and then directly mounted on a preparative TLC plate (1 mm silica gel plate per 0.4 mmol starting aryl halide). The reaction solvent was removed in a vigorous stream of air, and separation was achieved using petroleum ether / ethyl acetate (PE/EA 5:1 Volume/Volume) mixtures.

Said process was carried out according to the method described in the reference of S.L. Buchwald et al., J. Am. Chem. Soc. 2002, 124, 11684-11688. The respective part of the description is hereby incorporated by reference and forms part of the present disclosure.

### 4c. Preparation of compounds 18-II, 19-II and 20-II

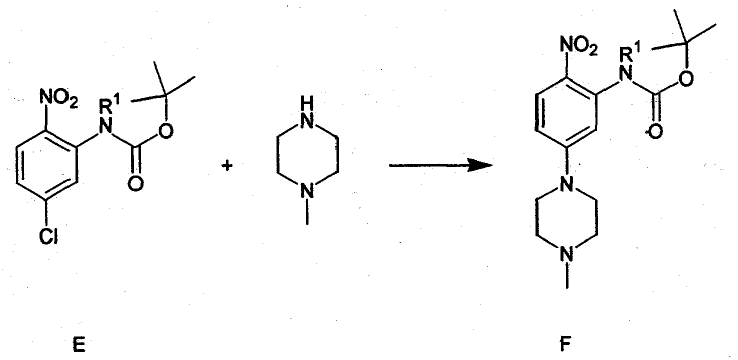

An oven-dried vial was subsequently charged with 0.1 eq. of a palladium source [Pd], 0.2 eq. (biph)P(*t*Bu)₂, 1.0 eq. arylhalide E and 1.4 eq. base. The vial was evacuated and purged with argon, and 1-methyl-piperazine (1.3 eq.) was added. The solvent was added to obtain a concentration of 1.0 M of the arylhalide E. The vial was sealed and heated to either 100°C in case of toluene as the solvent or 110°C in case of DME as the solvent in each case for 22 h. The reaction mixture was transferred into a flask using H₂O and MeOH and the total volume was reduced to less than 10 mL. Purification was achieved by preparative HPLC. In order to remove any ammonium formate derivative formed during preparative HPLC, the resulting product was taken up in chloroform, extracted twice with saturated aq. NaHCO₃, the combined aq. phases were re-extracted with chloroform, and the combined organic phases were dried over MgSO₄.

The different reaction conditions are listed in the following table 4.

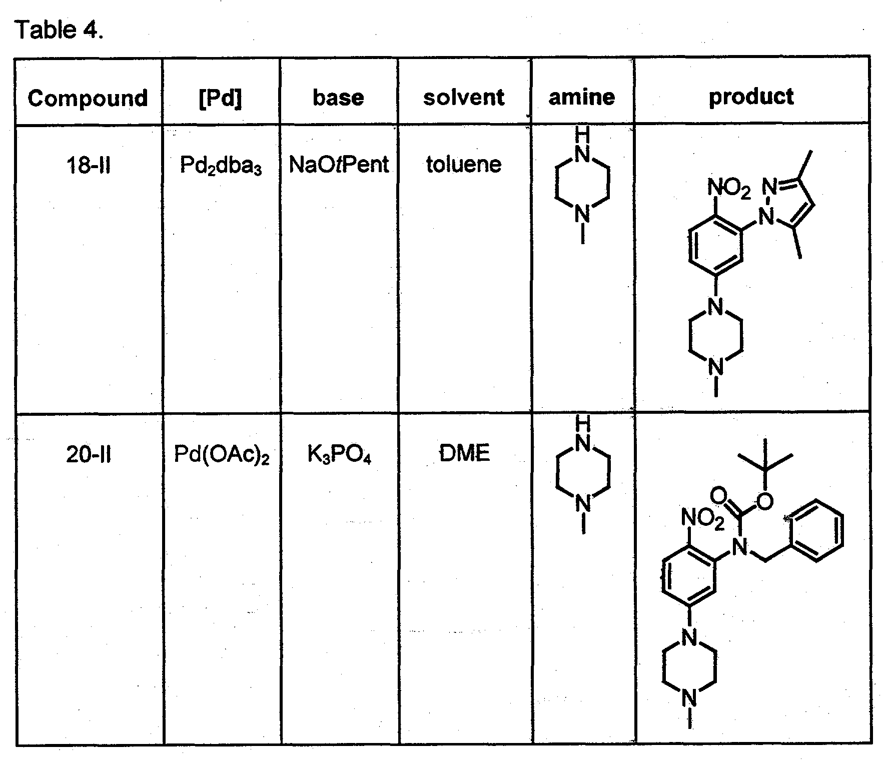

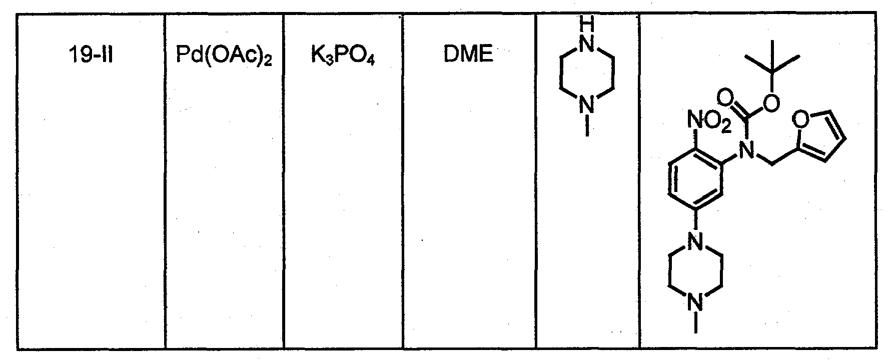

### 4d. Preparation of example compound 18

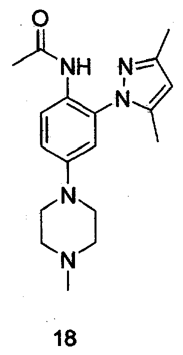

The compound 18-II was dissolved in THF, 0.1 eq. 5 % palladium on charcoal was added and the mixture was stirred in a hydrogen atmosphere at rt for 5 hours. The palladium on charcoal was removed by filtration and the filtrate was directly used in the next reaction.
The filtrate was treated with 1.0 eq. acetanhydride at rt for 5 hours. The reaction mixture was adjusted to pH 7-8 using aq. sat. NaHCO₃ and the product was extracted with DCM. The organic layer was dried over MgSO₄, concentrated and purified by prep. TLC to obtain 18.

### 4e. Preparation of example compounds 19 to 22

The compound 19-II or 20-II was dissolved in THF, 0.1 eq. 5 % palladium on charcoal was added and the mixture was stirred in a hydrogen atmosphere at rt for 5 hours. The palladium on charcoal was removed by filtration and the filtrate was directly used in the next reaction. The filtrate was directly treated with 4.0 M hydrochloric acid in dioxane and stirred overnight at rt. The mixture was neutralised using aq. sat. NaHCO₃ and extracted with ethyl acetate. The combined organic phases were dried over MgS04 and the solvent was removed in vacuo.

The crude material was dissolved in THF and treated with 1.0 eq. acetanhydride at rt for 5 hours. The reaction mixture was adjusted to pH 7-8 using aq. sat. NaHCO₃ and the product was extracted with DCM. The organic layer was dried over MgSO₄, concentrated and purified by prep. TLC to obtain 19 to 22.

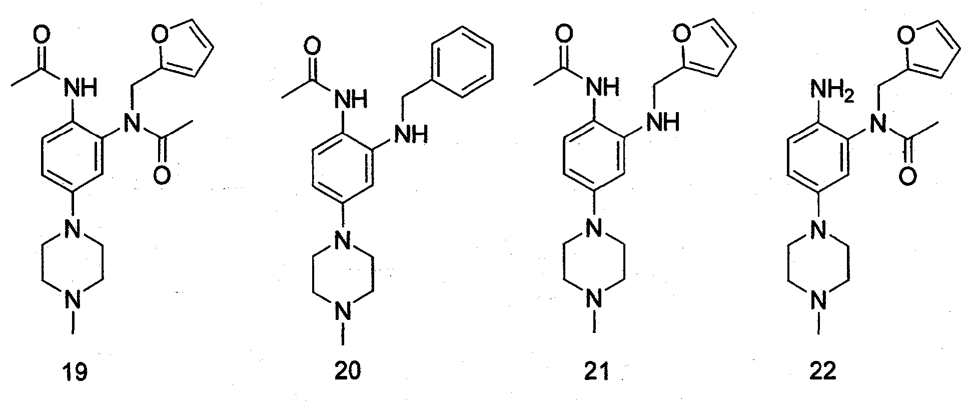

### 4f. Preparation of example compounds 24 and 25

The crude material as described under 4e. was dissolved in THF and treated with 1.0 eq. sulfonyl chloride at rt for 5 hours. The reaction mixture was adjusted to pH 7-8 using aq. sat. NaHCO₃ and the product was extracted with DCM. The organic layer was dried over MgSO₄, concentrated and purified by prep. TLC to obtain 24 or 25.

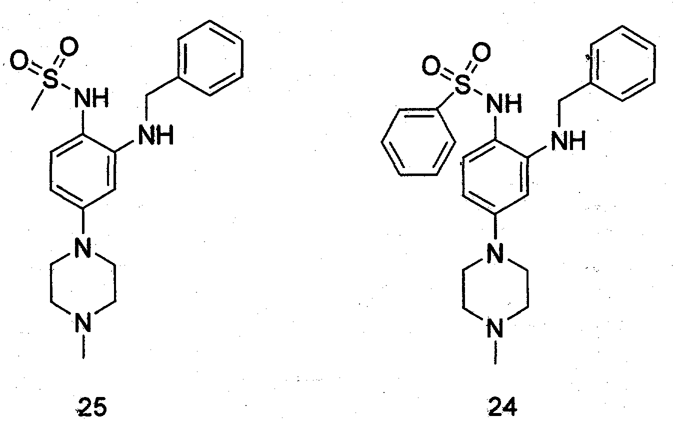

### 5. Preparation of example compounds 17, 23 and 27

The starting material 2-bromo-4-chloro-1-nitro-benzene was obtained by oxidation from 2-bromo-5-chloro-phenylamine according to a reference by A. McKillop et al., Tetrahedron 1987,42,1753.

### 5a. Preparation of compound 17-I

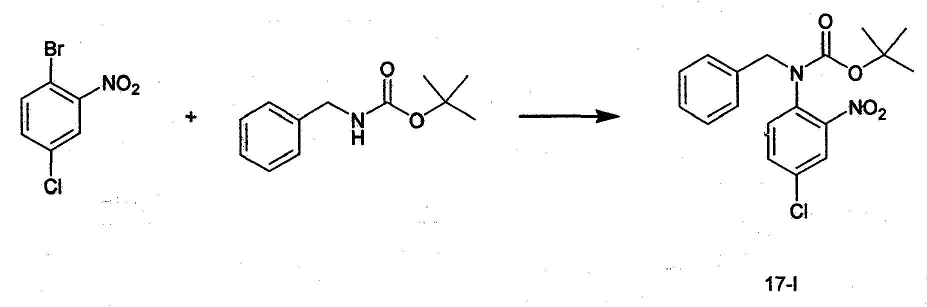

An oven-dried vial was subsequently charged with a palladium source [Pd], xantphos (1.5 eq. to Pd), 1.0 eq. 1-bromo-4-chloro-2-nitro-benzene, 1.1 eq. benzyl carbamic acid tert.-butyl ester and 1.4 eq. base. The tube was evacuated, purged with argon and dioxane was added to obtain a concentration of 1.0 M for 1-bromo-4-chloro-2-nitro-benzene. The vial was sealed and heated to 110°C for 21 h.
The reaction mixture was directly mounted on silica gel and separated using Flash Master II to obtain the desired product 17-1.

### 5b. Preparation of compound 17-II

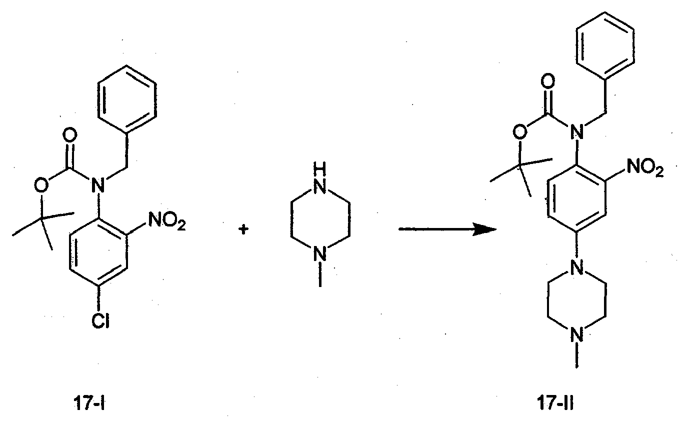

An oven-dried vial was subsequently charged with 0.1 eq. of palladium acetate, 0.2 eq. (biph)P(*t*Bu)₂, 1.0 eq. arylhalide 17-I and 1.4 eq. K₃PO₄. The vial was evacuated and purged with argon, and 1-methyl-piperazine (1.3 eq.) was added. DME was added to obtain a concentration of 1.0 M of the arylhalide 17-I. The vial was sealed and heated to 110°C for 22 h. The reaction mixture was transferred into a flask using H₂O and MeOH and the total volume was reduced to less than 10 mL. Purification was achieved by preparative HPLC. In order to remove any ammonium formate derivative formed during preparative HPLC, the resulting product was taken up in chloroform, extracted twice with saturated aq. NaHCO₃, the combined aq. phases were re-extracted with chloroform, and the combined organic phases were dried over MgSO₄.

### 5c. Preparation of example compounds 27

The compound 17-II was directly treated with 4.0 M hydrochloric acid in dioxane and stirred overnight at rt. The mixture was neutralised using aq. sat. NaHCO₃ and extracted with ethyl acetate. The combined organic phases were dried over MgSO₄ and the solvent was removed in vacuo. The product was obtained after purification by prep. TLC.

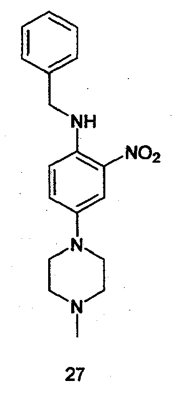

### 5d. Preparation of example compounds 17 and 23

The compound 17-II was dissolved in THF, 0.1 eq. 5 % palladium on charcoal was added and the mixture was stirred in a hydrogen atmosphere at rt for 5 hours. The palladium on charcoal was removed by filtration and the filtrate was directly used in the next reaction. The filtrate was directly treated with 4.0 M hydrochloric acid in dioxane and stirred overnight at rt. The mixture was neutralised using aq. sat. NaHCO₃ and extracted with ethyl acetate. The combined organic phases were dried over MgSO₄ and the solvent was removed in vacuo.

The crude material was dissolved in THF and treated with 1.0 eq. acetanhydride at rt for 5 hours. The reaction mixture was adjusted to pH 7-8 using aq. sat. NaHCO₃ and the product was extracted with DCM. The organic layer was dried over MgSO₄, concentrated and purified by prep. TLC to obtain 17 or 23.

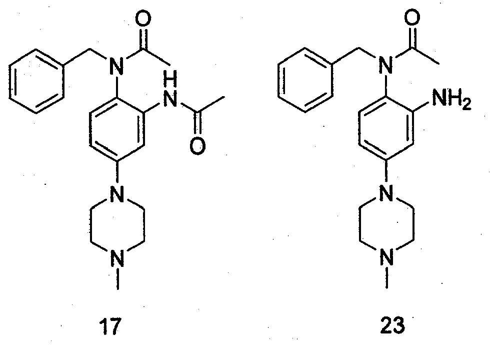

### Pharmacological data:

The binding of the substituted phenyl-piperazine compounds to the 5-HT₆ receptor was determined as described above.

The binding results for some of these compounds are given in the following table :

| **Compound according to example:** | **K**_{**i**} **(nM)** |
|---|---|
| 5 | 108,3 |
| 6 | 462 |
| 9 | 1284,2 |
| 10 | 401,8 |
| 11 | 409,4 |
| 23 | 164,0 |
| 24 | 60,2 |

## Claims

**1.** A substituted phenyl-piperazine compound of general formula I, 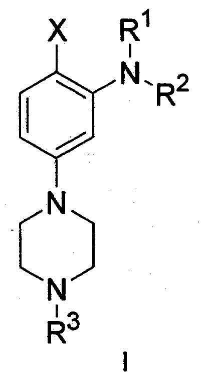 wherein
X represents -CN, -C(=O)-OH, -C(=O)-OR⁴, -O-R⁵, -NH₂, -NR⁶-C(=O)-R⁷, -NH-S(=O)₂-R⁸ or -NH-R⁹;
R¹ represents a hydrogen atom;
a saturated or unsaturated 3- to 9-membered cycloaliphatic radical, which may be unsubstituted or substituted with 1, 2 or 3 substituent(s) independently selected from the group consisting of oxo (=O), thioxo (=S), C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may contain1, 2 or 3 heteroatom(s) independently selected from the group consisting of nitrogen, oxygen and sulfur as ring member(s) and which is bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; or
a 5- to 10-membered aryl or heteroaryl radical, which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which is bonded via a linear or branched C₁₋₆ alkylene, C₂₋₆ alkenylene or C₂₋₆ alkinylene group which may contain 1, 2 or 3 heteroatom(s) independently selected from the group consisting of nitrogen, oxygen and sulfur as chain member(s) and which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH(C₁₋₅-alkyl) and -N(C₁₋₅-alkyl)₂ and whereby said heteroaryl radical contains 1, 2 or 3 heteroatom(s) independently selected from the group consisting of nitrogen, oxygen and sulfur as ring member(s);
R² represents a hydrogen atom or a -C(=O)-R¹⁰ moiety;
or
R¹ and R² together with the bridging nitrogen form a Nitro (NO₂)-group or
a 5- or 6-membered heteroaryl radical which may contain 1, 2 or 3 additional heteroatom(s) independently selected from the group consisting of nitrogen, oxygen and sulfur as ring member(s) and which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which may be condensed with a monocyclic ring system which may be unsubstituted or substituted with 1, 2 or 3 substituent(s) independently selected from the group consisting of oxo (=O), thioxo (=S), C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
whereby the ring of the ring system is 5- 6- or 7-membered and may contain 1, 2 or 3 heteroatom(s) independently selected from the group consisting of nitrogen, oxygen and sulfur;
R³ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂;
R⁴ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂;
R⁵ represents a linear or branched, saturated or unsaturated C₁₋₁₀ aliphatic radical which may be unsubstituted or substituted with 1, 2 or 3 substituerit(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂; or
a 5- to 10-membered aryl or heteroaryl radical which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂ and whereby the heteroaryl radical contains 1, 2 or 3 heteroatom(s) independently selected from the group consisting of nitrogen, oxygen and sulfur as ring member(s);
R⁶ represents a hydrogen atom or
a 5- to 10-membered aryl or heteroaryl radical which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂ and whereby the heteroaryl radical contains 1, 2 or 3 heteroatom(s) independently selected from the group consisting of nitrogen, oxygen and sulfur as ring member(s);
R⁷ represents a linear or branched, saturated or unsaturated C₁₋₁₀ aliphatic radical which may be unsubstituted or substituted with 1, 2 or 3 substituent(s) independently selected from the group consisting off, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂;
R⁸ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂; or
a 5- to 10-membered aryl or heteroaryl radical which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂ and whereby the heteroaryl radical contains 1, 2 or 3 heteroatom(s) independently selected from the group consisting of nitrogen, oxygen and sulfur as ring member(s);
R⁹ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂; or
a 5- to 10-membered aryl radical which may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, - SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂₋phenyl, phenyl, phenoxy and benzyl and which maybe bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, - OH, -SH, -NH₂, -O-C₁₋₅-alkyl, -S-C₁₋₅-alkyl, -NH(C₁₋₅-alkyl) and -N(C₁₋₅-alkyl)₂;
and
R¹⁰ represents a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, - OCF₃, -SCF₃, -OH, -SH, -NH₂, -O-C₁₋₅-alkyl, -S-C₁₋₅-alkyl, -NH(C₁₋₅-alkyl) and - N(C₁₋₅-alkyl)₂;
optionally in form of one of its stereoisomers, preferably enantiomers or diasteromers, a racemate or in form of a mixture of at least two of its stereoisomers, preferably enantiomers and/or diastereomers, in any mixing ratio, or a physiologically acceptable salt thereof, or a corresponding solvate thereof,
with the provisos
that R¹ and R² do not both represent a hydrogen atom if X represents an -NH₂ group and
that R¹ does not represent a hydrogen atom if X represents an -NH-S(=O)₂-R⁸ group.

**2.** A compound according to claim 1, **characterized in that**
R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thioxo (=S), C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; or
an aryl or heteroaryl radical selected from the groupconsisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, - (CH₂)-(CH₂)-, -(CH₂)-(CH₂)-(CH₂)-, -O-(CH₂)-(CH₂)- or -(CH₂)-(CH₂)-O-group and/or may be unsubstituted or substituted with 1, 2 or 3 substituent(s) independently selected from the group consisting of C₁₋₅-alkyl, -O-C₁₋₅-alkyl, - S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S-(=O)₂-phenyl, phenyl, phenoxy and benzyl;
preferably R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -0-C₂H₅, -O-CH₂-CH₂-CH₃, -O-CH(CH₃)₂, -O-C(CH₃)₃, -S-CH₃, -S-C₂H₅, -S-CH₂₋CH₂-CH₃, -S-CH(CH₃)₂, -S-C(CH₃)₃, -O(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃,-C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ and -S(=O)₂-CH₃; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, - (CH₂)-(CH₂)-, -(CH₂)-(CH₂)-(CH₂)-, -O-(CH₂)-(CH₂)- or -(CH₂)-(CH₂)-O-group and/or may be unsubstituted or substituted with 1, 2 or 3 substituent(s) independently selected from the group-consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF3, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH=CH₃, -C(=O)-NH-C₂H₅, -C(=O)-N(CH₃)₂, -C(=O)-N(C₂H₅)₂ and -S(=O)₂-CH₃,
more preferably R¹ represents
a hydrogen atom; or
an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH.

**3.** A compound according to claim 1 or 2, **characterized in that** R¹ and R² together with the bridging nitrogen atom form a nitro group; or a moiety selected from the group consisting of 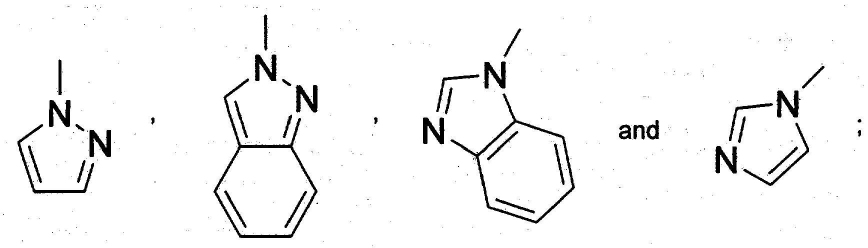 whereby each of these afore mentioned cyclic moieties may be unsubstituted substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅₋alkyl, -C(=O)-O-C₁₋₅-alkyl, -O-C(=O)-C₁₋₅-alkyl, F, Cl, Br, I, -CN, -CF₃, -OCF₃, - SCF₃, -OH, -SH, -NH₂, -NH(C₁₋₅-alkyl), -N(C₁₋₅-alkyl)₂, -NO₂, -CHO, -CF₂H, - CFH₂, -C(=O)-NH₂, -C(=O)-NH(C₁₋₅-alkyl), -C(=O)-N(C₁₋₅-alkyl)₂, -S(=O)₂-C₁₋₅₋alkyl, -S(=O)₂-phenyl, phenyl, phenoxy and benzyl;
preferably R¹ and R² together with the bridging nitrogen atom form a nitro group or a moiety selected from the group consisting of 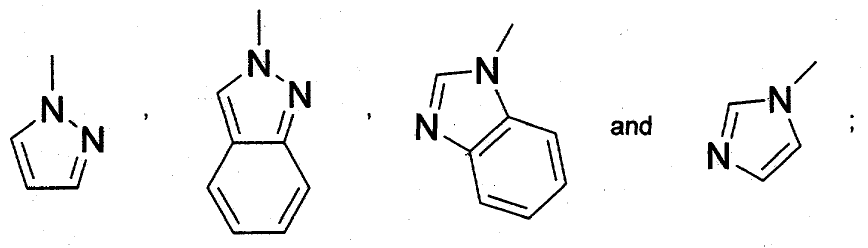 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,
more preferably R¹ and R² together with the bridging nitrogen atom form a nitro group or a moiety selected from the group consisting of 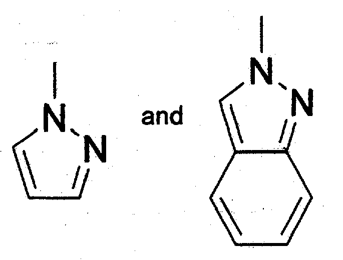 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,

**4.** A compound according to one or more of claims 1 to 3, **characterized in that** R³ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and -SH;
preferably R³ represents an alkyl radical selected from the group consisting of methyl, ethyl, n-propyl, isopropyl, n-butyl, sec-butyl, isobutyl and tert-butyl;
more preferably R³ represents a methyl or ethyl radical.

**5.** A compound according to one or more of claims 1 to 4, **characterized in that** R⁴ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and -SH;
preferably R⁴ represents an alkyl radical selected from the group consisting of methyl, ethyl, n-propyl, isopropyl, n-butyl, sec-butyl, isobutyl and tert-butyl;
more preferably R⁴ represents a methyl or ethyl radical.

**6.** A compound according to one or more of claims 1 to 5, **characterized in that** R⁵ represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, - OH and SH;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1,2 or 3}-group and/or may be unsubstituted or substituted with 1, 2 or 3 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁵ represents
an alkyl radical selected from the group consisting of methyl, ethyl, n-propyl, isopropyl, n-butyl, sec-butyl, isobutyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1,2 or 3}-group and/or may be unsubstituted or substituted with 1, 2 or 3 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
more preferably R⁵ represents
an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH.

**7.** A compound according to one or more of claims 1 to 6, **characterized in that** R⁶ represents
a hydrogen atom or
an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁶ represents
a hydrogen atom or
an aryl radical selected from the group consisting of phenyl and naphthyl, whereby said aryl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br,-CF₃, -OCF₃, -OH and -SH;
more preferably R⁶ represents a hydrogen atom, or
a phenyl radical, whereby said phenyl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br,-CF₃, -OCF₃, -OH and -SH.

**8.** A compound according to one or more of claims 1 to 7, **characterized in that** R⁷ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and -SH;
preferably R⁷ represents an alkyl radical selected from the group consisting of methyl, ethyl, n-propyl, isopropyl, n-butyl, sec-butyl, isobutyl and tert-butyl;
more preferably R⁷ represents a methyl or ethyl radical.

**9.** A compound according to one or more of claims 1 to 8, **characterized in that** R⁸ represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -S-CF₃, - OH and SH; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1,2} or ₃-group and/or may be unsubstituted or substituted with 1, 2 or 3 substituent(s) independently selected from the group consisting of C₁₋₅-alkyl,-O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁸ represents
an alkyl radical selected from the group consisting of methyl, ethyl, n-propyl, isopropyl, n-butyl, sec-butyl, isobutyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1,2 or 3}-group and/or may be unsubstituted or substituted with 1, 2 or 3 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
more preferably R⁸ represents
an alkyl radical selected from the group consisting of methyl, ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH₂)-, or -(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂₋CH₃, -O-CH(CH₃)₂, -O-C(CH₃)₃, F, Cl, Br, -CN, -CF₃, -OCF₃, -OH and -SH.

**10.** A compound according to one or more of claims 1 to 9, **characterized in that** R⁹ represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, - OH and SH;
or an aryl radical selected from the group consisting of phenyl and naphthyl, whereby said aryl radical may be bonded via a -(CH₂)_{1.2 or 3}-group and/or may be unsubstituted or substituted with 1, 2 or 3 substituent(s) independently selected from the group consisting of-C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
preferably R⁹ represents
an alkyl radical selected from the group consisting of methyl; ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH₂)_{1,2} or ₃-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋5-alkyl)₂, -S(=O)₂-C₁₋₅-alkyl, -S(=O)₂-phenyl, phenyl, phenoxy and benzyl;
more preferably R⁹ represents
an alkyl radical selected from the group consisting of methyl, ethyl, n-propyl, isopropyl, n-butyl, sec-butyl, isobutyl and tert-butyl;
or an aryl radical selected from the group consisting of phenyl and naphthyl, whereby said aryl radical may be bonded via a -(CH₂)-, -(CH₂)-(CH₂)- or - (CH₂)-(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, -CN, -CF₃, - OCF₃, -OH and -SH.

**11.** A compound according to one or more of claims 1 to 10, **characterized in that** R¹⁰ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, - CF₃, -OCF₃, -SCF₃, -OH and -SH;
preferably R¹⁰ represents an alkyl radical selected from the group consisting of methyl, ethyl, n-propyl, isopropyl, n-butyl, sec-butyl, isobutyl and tert-butyl;
more preferably R¹⁰ represents a methyl or ethyl radical.

**12.** A compound according to one or more of claims 1 to 11, **characterized in that**
X represents -CN, -C(=O)-OH, -C(=O)-OR⁴, -O-R⁵, -NH₂, -NR⁶-C(=O)-R⁷, - NH-S(=O)₂-R⁸ or -NH-R⁹;
R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thioxo (=S), C₁₋₅-alkyl, -O-C₁₋₅₋alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, =OH, -SH, -NH₂, - NH(C₁₋₅-alkyl), -N(C₁₋₅-alkyl)₂, -NO₂, -CHO, -CF₂H, -CFH₂, -C(=O)-NH₂,-C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo(2,1-b]thiazolyl, whereby said aryl or heteroaryl radical is bonded via a -(CH₂)-, - (CH₂)-(CH₂)-, -(CH₂)-(CH₂)-(CH₂)-, -O-(CH₂)-(CH₂)- or -(CH₂)-(CH₂)-O-group and/or may be unsubstituted or substituted with 1, 2 or 3 substituent(s) independently selected from the group consisting of C₁₋₅-alkyl, -O-C₁₋₅-alkyl, - S-C₁₋₅-alkyl, -C(=O)-OH, -C(=O)-C₁₋₅-alkyl, -C(=O)-O-C₁₋₅-alkyl, -O-C(=O)-C₁₋₅₋alkyl, F, Cl, Br, I, -CN, -CF₃, -OCF₃, -SCF₃, -OH, -SH, -NH₂, -NH(C₁₋₅-alkyl), - N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R² represents a hydrogen atom or a -C(=O)-R¹⁰ moiety;
R¹ and R² together with the bridging nitrogen atom form a nitro group; or a moiety selected from the group consisting of 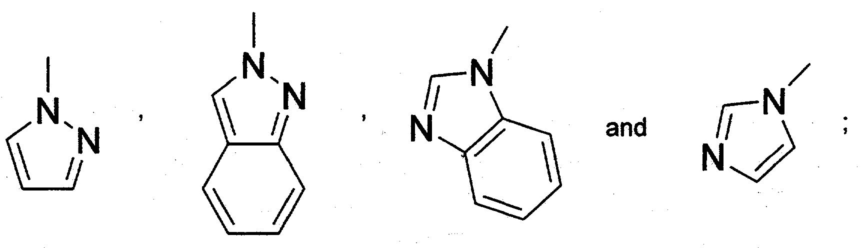 whereby each of these afore mentioned cyclic moieties may be unsubstituted or substituted with 1, 2 or 3 substituent(s) independently selected from the group consisting of C₁₋₅-alkyl, -O-C₁₋₅-alkyl, -S-C₁₋₅-alkyl, -C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, - OCF₃, -SCF₃, -OH, -SH, -NH₂, -NH(C₁₋₅-alkyl), -N(C₁₋₅-alkyl)₂, -NO₂, -CHO, - CF₂H, -CFH₂, -C(=O)-NH₂, -C(=O)-NH(C₁₋₅-alkyl), -C(=O)-N(C₁₋₅-alkyl)₂, - S(=O)₂-C₁₋₅-alkyl, -S(=O)₂-phenyl, phenyl, phenoxy and benzyl;
R³ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and -SH;
R⁴ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and -SH;
R⁵ represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, CI, Br, I, -CN, -CF₃, -OCF₃, -SCF₃, - OH and SH;
or an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1,2} or ₃-group and/or may be unsubstituted or substituted with 1, 2 or 3 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁶ represents
a hydrogen atom or
an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-O(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁷ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, -CF₃, -OCF₃, -SCF₃, -OH and -SH;
R⁸ represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F, Cl, Br, I, -CN, -CF₃, -OCF₃, -SCF₃, - OH and SH; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1,2 or 3}-group and/or may be unsubstituted or substituted with 1, 2 or 3 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R9 represents
a linear or branched C₁₋₁₀ alkyl radical, which may be unsubstituted or substituted with 1, 2 or 3 substituent(s) independently selected from the group consisting of -O-C₁₋₅-alkyl, -S-C₁₋₅-alkyl, F; Cl, Br, I, -CN, -CF₃, -OCF₃, -SCF₃, - OH and SH;
or an aryl radical selected from the group consisting of phenyl and naphthyl, whereby said aryl radical may be bonded via a -(CH₂)_{1,2} or ₃-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R¹⁰ represents a linear or branched C₁₋₁₀ alkyl radical that may be unsubstituted or substituted with 1, 2 or 3 substituent(s) independently selected from the group consisting of -O-C₁₋₅-alkyl, -S-C₁₋₅-alkyl, F, Cl, Br, I, - CF3, -OCF₃, -SCF₃, -OH and -SH.

**13.** A compound according to one or more of claims 1 to 12, **characterized in that**
X represents -CN, -C(=O)-OH, -C(=O)-OR⁴, -O-R⁵, -NH₂, -NR⁶-C(=O)-R⁷,-NH-S(=O)₂-R⁸ or -NH-R⁹;
R¹ represents
a hydrogen atom;
a (hetero)cycloaliphatic radical selected from the group consisting of cyclopropyl, cyclobutyl, cyclopentyl, cyclohexyl, cycloheptyl, cyclooctyl, cyclononyl, imidazolidinyl, aziridinyl, azetidinyl, pyrrolidinyl, piperidinyl, morpholinyl, thiomorpholinyl, piperazinyl, pyrazolidinyl and azepanyl, whereby said (hetero)cycloaliphatic radical is bonded via a -(CH₂)_{1, 2 or 3}- group and/or may be unsubstituted or substituted with 1, 2 or 3 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂₋CH₂-CH₃, -S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, - C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH3)₂, -N(C₂H₅)₂, - NO₂, -CHO, -CF₂H, -CFH₂, -C(=O)-NH₂, -C(=O)-NH-CH₃, -C(=O)-NH-C₂H₅, - C(=O)-N(CH₃)₂, -C(=O)-N(C₂H₅)₂ and -S(=O)₂-CH₃; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical is bonded via a -(CH₂)-, - (CH₂)-(CH₂)-, -(CH₂)-(CH₂)-(CH₂)-, -O-(CH₂)-(CH₂)- or -(CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -O-CH₂-CH₂-CH₃, -O-CH(CH₃)₂, -O-C(CH₃)₃, -S-CH₃, -S-C₂H₅, -S-CH₂-CH₂-CH₃, - S-CH(CH₃)₂, -S-C(CH₃)₃, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, - C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NH-CH₃, -NH-C₂H₅, -NH-CH₂-CH₂-CH₃, -NH-CH(CH₃)₂, -NH-C(CH₃)₃, -N(CH₃)₂, -N(C₂H₅)₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂ and -S(=O)₂-CH₃,
R² represents a hydrogen atom or a -C(=O)-R¹⁰ moiety;
preferably R¹ and R² together with the bridging nitrogen atom form a nitro group or a moiety selected from the group consisting of 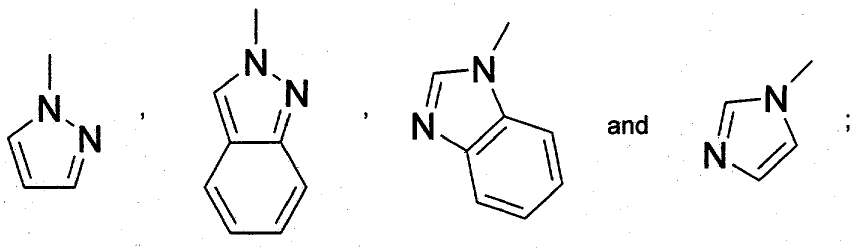 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,
R³ represents an alkyl radical selected from the group consisting of methyl, ethyl, n-propyl, isopropyl, n-butyl, sec-butyl, isobutyl and tert-butyl;
R⁴ represents an alkyl radical selected from the group consisting of methyl, ethyl, n-propyl, isopropyl, n-butyl, sec-butyl, isobutyl and tert-butyl;
R⁵ represents
an alkyl radical selected from the group consisting of methyl, ethyl, n-propyl, isopropyl, n-butyl, sec-butyl, isobutyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may bebonded via a - (CH₂)_{1,2 or 3}-group and/or may be unsubstituted or substituted with 1, 2 or 3 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂-, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁶ represents
a hydrogen atom or
an aryl radical selected from the group consisting of phenyl and naphthyl, whereby said aryl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br,-CF₃, -OCF₃, -OH and -SH,
R⁷ represents an alkyl radical selected from the group consisting of methyl, ethyl, n-propyl, isopropyl, n-butyl, sec-butyl, isobutyl and tert-butyl;
R⁸ represents
an alkyl radical selected from the group consisting of methyl, ethyl, n-propyl, isopropyl, n-butyl, sec-butyl, isobutyl and tert-butyl; or
an aryl or heteroaryl radical selected from the group consisting of phenyl, naphthyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridinyl, pyridazinyl, pyrimidinyl, pyrazinyl, quinolinyl, isoquinolinyl, benzo[b]furanyl, benzo[b]thiophenyl, benzothiadiazolyl and imidazo[2,1-b]thiazolyl, whereby said aryl or heteroaryl radical may be bonded via a - (CH₂)_{1,2 or 3}-group and/or may be unsubstituted or substituted with 1, 2 or 3 substituent(s) independently selected from the group consisting of C₁₋₅-alkyl, - O-C₁₋₅-alkyl, -S-C₁₋₅-alkyl, -C(=O)-OH, -C(=O)-C₁₋₅-alkyl, -C(=O)-O-C₁₋₅-alkyl, - O-C(=O)-C₁₋₅-alkyl, F, Cl, Br, I, -CN, -CF₃, -OCF₃, -SCF₃, -OH, -SH, -NH₂, - NH(C₁₋₅-alkyl), -N(C₁₋₅-alkyl)₂, -NO₂, -CHO, -CF₂H, -CFH₂, -C(=O)-NH₂, - C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R⁹ represents
an alkyl radical selected from the group consisting of methyl, ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH₂)_{1,2 or 3}-group and/or may be unsubstituted or substituted with 1, 2 or 3 substituent(s) independently selected from the group consisting of C₁₋₅-alkyl, -O-C₁₋₅-alkyl, -S-C₁₋₅-alkyl, - C(=O)-OH, -C(=O)-C₁₋₅-alkyl, -C(=O)-O-C₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=O)-NH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl;
R¹⁰ represents an alkyl radical selected from the group consisting of methyl, ethyl, n-propyl, isopropyl, n-butyl, sec-butyl, isobutyl and tert-butyl.

**14.** A compound according to one or more of claims 1 to 13, **characterized in that**
X represents -CN, -C(=O)-OH, -C(=O)-OR⁴, -O-R⁵, -NH₂, -NR⁶-C(=O)-R⁷, - NH-S(=O)₂-R⁸ or -NH-R⁹;
R¹ represents
a hydrogen atom; or
an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, -CN, -CF₃, -OCF₃, -OH and -SH.
R² represents a hydrogen atom or a -C(=O)-R¹⁰ moiety;
R¹ and R² together with the bridging nitrogen atom form a nitro group or moiety selected from the group consisting of 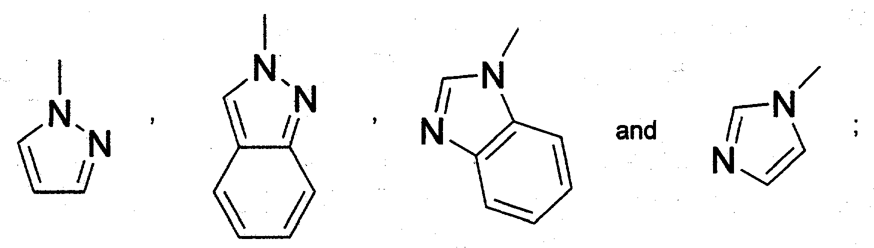 whereby each of these afore mentioned cyclic moieties may be unsubstituted or substituted with 1, 2 or 3 substituent(s) independently selected from the group consisting of methyl, ethyl, n-propyl, isopropyl, n-butyl, tert-butyl, sec-butyl, isobutyl, F, Cl, Br, I, -CN and -CF₃,
R3 represents a methyl or ethyl radical;
R⁴ represents a methyl or ethyl radical;
R⁵ represents
an alkyl radical selected from the group consisting of methyl, ethyl, n-propyl, isopropyl, n-butyl, sec-butyl, isobutyl and tert-butyl;
or an aryl or heteroaryl radical selected from the group consisting of phenyl, naphthyl, furyl (furanyl) and thienyl (thiophenyl), whereby said aryl or heteroaryl radical is bonded via a -(CH₂)-, -(CH₂)-(CH₂)- or -(CH₂)-(CH₂)-(CH₂)- group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂-CH₃, -O-CH(CH₃)₂, -O-C(CH₃)₃, F, Cl, Br, -CN; -CF₃, - OCF₃, -OH and -SH;
R⁶ represents a hydrogen atom, or
a phenyl radical, whereby said phenyl radical may be bonded via a -(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br,-CF₃, -OCF₃, -OH and -SH;
R⁷ represents a methyl or ethyl radical;
R⁸ represents
an alkyl radical selected from the group consisting of methyl, ethyl, n-propyl, isopropyl, n-butyl, sec-butyl, isobutyl and tert-butyl; or
an aryl radical selected from the group consisting of phenyl and naphthyl, whereby said aryl radical may be bonded via a -(CH₂)-, or -(CH₂)-(CH₂)-group and/or may be unsubstituted or substituted with 1, 2 or 3 substituent(s) independently selected from the group consisting of methyl, ethyl, n-propyl, isopropyl, n-butyl, tert-butyl, sec-butyl, isobutyl, -O-CH₃, -O-C₂H₅, -O-CH₂-CH₂₋CH₃, -O-CH(CH₃)₂, -O-C(CH₃)₃, F, Cl, Br, -CN, -CF₃, -OCF₃, -OH and -SH,
R⁹ represents
an alkyl radical selected from the group consisting of methyl, ethyl, n-propyl, isopropyl, n-butyl, sec-butyl, isobutyl and tert-butyl;
or an aryl radical selected from the group-consisting of phenyl and naphthyl, whereby said aryl radical may be bonded via a -(CH₂)-, -(CH₂)-(CH₂)- or - (CH₂)-(CH₂)-(CH₂)-group and/or may be unsubstituted or substituted with 1,2 or 3 substituent(s) independently selected from the group consisting of methyl, ethyl, n-propyl, isopropyl, n-butyl, tert-butyl, sec-butyl, isobutyl, -O-CH₃, -0-C₂H₅, -O-CH₂-CH₂-CH₃, -O-CH(CH₃)₂, -O-C(CH₃)₃, F, Cl, Br, -CN, -CF₃, - OCF₃, -OH and -SH;
R¹⁰ represents a methyl or ethyl radical.

**15.** A compound according to one or more of claims 1 to 14, **characterized in that**
X represents -CN, -C(=O)-OH, -C(=O)-OR⁴, -O-R⁵, -NH₂, -NR⁶-C(=O)-R⁷, - NH-S(=O)₂-R⁸ or -NH-R⁹;
R¹ represents
a hydrogen atom; or
an unsubstituted aryl or heteroaryl radical selected from the group consisting of phenyl, furyl (furanyl) and thienyl (thiophenyl), whereby said aryl or heteroaryl radical is bonded via a -(CH₂)-, -(CH₂)-(CH₂)-, -(CH₂)-(CH₂)-(CH₂)-, -O-(CH₂)-(CH₂)-, or -(CH₂)-(CH₂)-O-group,
R² represents a hydrogen atom or a -C(=O)-R¹⁰ moiety;
or
R¹ and R² together with the bridging nitrogen atom form a nitro group or a cyclic moiety of the following formula: 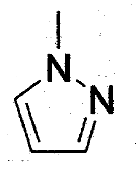 whereby said cyclic moiety may be substituted with 1, 2 or 3 substituent(s) independently selected from the group consisting of methyl, ethyl, n-propyl, isopropyl, n-butyl, isobutyl, sec-butyl and tert-butyl;
R³ represents a methyl or ethyl radical;
R⁴ represents a methyl or ethyl radical;
R⁵ represents a methyl radical, an ethyl radical; or an unsubstituted phenyl radical, which may be bonded via a -(CH₂)-group;
R⁶ represents a hydrogen atom or an unsubstituted benzyl radical;
R⁷ represents a methyl or ethyl radical;
R⁸ represents a methyl or ethyl radical; or an unsubstituted phenyl radical;
R⁹ represents an unsubstituted benzyl radical;
and
R¹⁰ represents a methyl or ethyl radical.

**16.** A compound according to one or more of claims 1 to 15 selected from the group consisting of
[1] 4-(4-Methyl-piperazin-1-yl)-2-phenethylamino-benzoic acid,
[2] 2-[(Furan-2-ylmethyl)-amino]-4-(4-methyl-piperazin-1-yl)-benzonitrile,
[3] 2-[(Furan-2-ylmethyl)-amino]-4-(4-methyl-piperazin-1-yl)-benzoic acid,
[4] 2-Benzylamino-4-(4-methyl-piperazin-1-yl)-benzoic acid methyl ester,
[5] 2-Benzylamino-4-(4-methyl-piperazin-1-yl)-benzonitrile,
[6] 4-(4-Methyl-piperazin-1-yl)-2-phenethylamino-benzoic acid methyl ester,
[7] 2-[(Furan-2-ylmethyl)-amino]-4-(4-methyl-piperazin-1-yl)-benzoic acid methyl ester,
[8] 2-Benzylamino-4-(4-methyl-piperazin-1-yl)-benzoic acid,
[9] [2-Benzyloxy-5-(4-methyl-piperazin-1-yl)-phenyl]-phenethyl-amine,
[10] [2-Benzyloxy-5-(4-methyl-piperazin-1-yl)-phenyl]-furan-2-yl-methyl amine,
[11] Benzyl-[2-methoxy-5-(4-methyl-piperazin-1-yl)-phenyl]-amine,
[12] [2-Methoxy-5-(4-methyl-piperazin-1-yl)-phenyl]-phenethyl-amine,
[13] Furan-2-ylmethyl-[2-methoxy-5-(4-methyl-piperazin-1-yl)-phenyl]-amine,
[14] Benzyl-[2-benzyloxy-5-(4-methyl-piperazin-1-yl)-phenyl]-amine,
[15] N-[2-Acetyl-(2-phenoxyethyl)-amino]-4-(4-methyl-piperazin-1-yl)-phenyl]-acetamide,
[16] N-[4-(4-Methyl-piperazin-1-yl)-2-(2-phenoxy-ethylamino)-phenyl]-acetamide,
[17] N-[2-(Acetyl-amino)-4-(4-methyl-piperazin-1-yl)-phenyl]-N-benzyl-acetamide,
[18] N-[2-(3,5-Dimethyl-pyrazol-1-yl)-4-(4-methyl-piperazin-1-yl)-phenyl]-acetamide,
[19] N-[2-(Acetyl-furan-2-ylmethyl-amino)-4-(4-methyl-piperazin-1-yl)-phenyl]-acetamide,
[20] N-[2-Benzylamino-4-(4-methyl-piperazin-1-yl)-phenyl]-acetamide,
[21] N-[2-[Furan-2-ylmethyl)-amino]-4-(4-methyl-piperazin-1-yl)-phenyl]-acetamide,
[22] N-[2-Amino-5-(4-methyl-piperazin-1 -yl)-phenyl]-N-furan-2-ylmethyl-acetamide,
[23] N-[2-Amino-4-(4-methyl-piperazin-1-yl)-phenyl]-N-benzyl-acetamide,
[24] N-[2-Benzylamino-4-(4-methyl-piperazin-1-yl)-phenyl]-benzenesulfonamide,
[25] N-[2-Benzylamino-4-(4-methyl-piperazin-1-yl)-phenyl]-methansulfonamide,
[26] 2-Benzyloxy-5-(4-methyl-piperazin-1-yl)-phenylamine,
[27] Benzyl-[4-(4-methyl-piperazin-1-yl)-2-nitro-phenyl]-amine and
[28] 2-Cyano-(5-piperazin-1-yl-methyl)-2-phenoxy-ethylamine
optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof.

**17.** Process for the preparation of a compound according to one or more of claims 1 to 16, **characterized in that** at least one substituted benzene compound of general formula II, 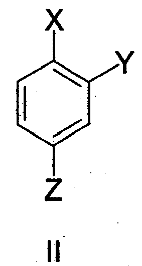 wherein X represents -CN, -C(=O)-OR⁴, -O-R⁵ or -NO₂, R⁴ and R⁵ have the meaning according to one or more of claims 1 to 16, Y represents a chlorine atom, and Z represents a bromine or iodine atom; is reacted with at least one piperazine compound of general formula III, 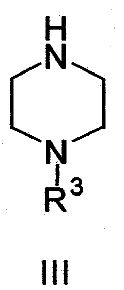 wherein R³ has the meaning according to one or more of claims 1 to 16, in a suitable reaction medium, preferably in the presence of at least one catalyst and/or at least one auxiliary agent and/or at least one base to yield a compound of general formula IV, 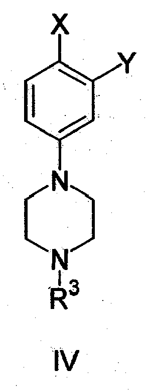 wherein X represents -CN, -C(=O)-OR⁴, -O-R⁵ or -NO₂, R³, R⁴ and R⁵ have the meaning according to one or more of claims 1 to 16 and Y represents a chlorine atom; which is optionally purified and/or isolated, and the compound of general formula IV is reacted with at least one compound of general formula V, 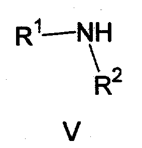 wherein R¹ and R² have the meaning according to one or more of claims 1 to 16 or one of them represents a protecting group, preferably a -C(=O)-O-C(CH₃)₃ group, in a suitable reaction medium, preferably in the presence of at least one catalyst and/or at least one auxiliary agent and/or at least one base to yield a compound of general formula VI, 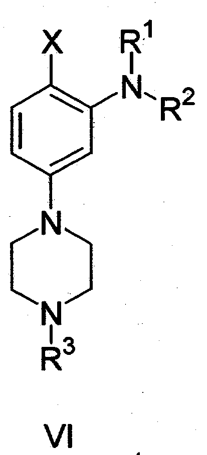 wherein X represents -CN, -C(=O)-OR⁴, -O-R⁵ or -NO₂, R¹, R² have the meaning according to one or more of claims 1 to 16 or one of them represents a protecting group, preferably -C(=O)-O-C(CH₃)₃ and R³, R⁴ and R⁵ have the meaning according to one or more of claims 1 to 16, said compound of general formula VI being optionally purified and/or isolated,
or at least one substituted benzene compound of general formula Ila, 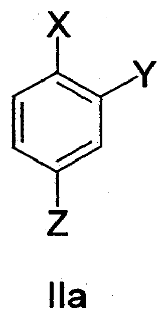 wherein X represents -CN, -C(=O)-OR⁴, -O-R⁵ or -NO₂, R⁴ and R⁵ have the meaning according to one or more of claims 1 to 16, Z represents a chlorine atom, Y represents a bromine or iodine atom, is reacted with at least one compound of general formula V, 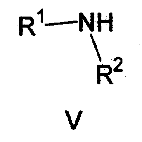 wherein R¹ and R² have the meaning according to one or more of claims 1 to 16 or one of them represents a protecting group, preferably a -C(=O)-O-C(CH₃)₃ group, in a suitable reaction medium, preferably in the presence of at least one catalyst and/or at least one auxiliary agent and/or at least one base to yield a compound of general formula VII, 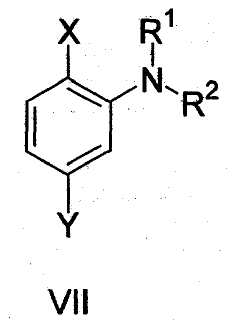 wherein X represents -CN, -C(=O)-OR⁴, -O-R⁵ or -NO₂, R¹ and R² have the meaning according to one or more of claims 1 to 16 or one of them represents a protecting group, preferably a -C(=O)-O-C(CH₃)₃ group, R⁴ and R⁵ have the meaning according to one or more of claims 1 to 16, and Y represents a chlorine atom; said compound of general formula being optionally purified and/or isolated, and the compound of general formula VII is reacted with at least one compound of general formula III, 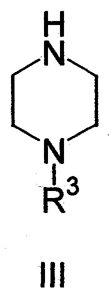 wherein R³ has the meaning according to one or more of claims 1 to 16, in a suitable reaction medium, preferably in the presence of at least one catalyst and/or at least one auxiliary agent and/or at least one base to yield a compound of general formula VI, 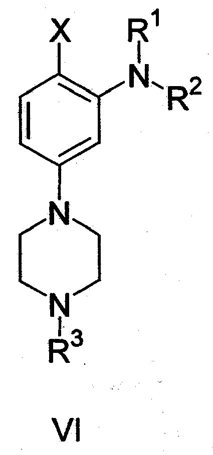 wherein X represents -CN, -C(=O)-OR⁴, -O-R⁵ or -NO₂, R¹ and R² have the meaning according to one or more of claims 1 to 16 or one of them represents a protecting group, preferably a -C(=O)-O-C(CH₃)₃ group, and R³, R⁴ and R⁵ have the meaning according to one or more of claims 1 to 16, and said compound of general formula VI is optionally purified and/or isolated,
or
at least one substituted benzene compound of general formula VIII, 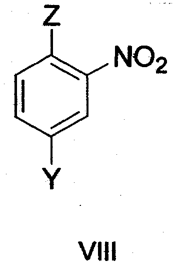 wherein Z represents bromine or iodine and Y represents chlorine, is reacted with at least one compound of general formula IX, 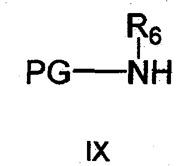 wherein R⁶ has the meaning according to one or more of claims 1 to 16 and PG represents a protecting group, preferably a-C(=O)-O-C(CH₃)₃ group, in a suitable reaction medium, preferably in the presence of at least one catalyst and/or at least one auxiliary agent and/or at least one base to yield a compound of general formula XI, 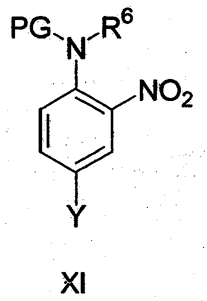 wherein R⁶ has the meaning according to one or more of claims 1 to 16, PG represents a protecting group, preferably a-C(=O)-O-C(CH₃)₃ group and Y represents chlorine; which is optionally purified and/or isolated, and the compound of general formula XI reacted with at least one compound of general formula III, 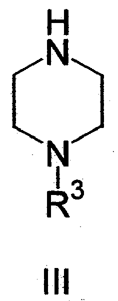 wherein R³ has the meaning according to one or more of claims 1 to 16, in a suitable reaction medium, preferably in the presence of at least one catalyst and/or at least one auxiliary agent and/or at least one base to yield a compound of general formula XII, 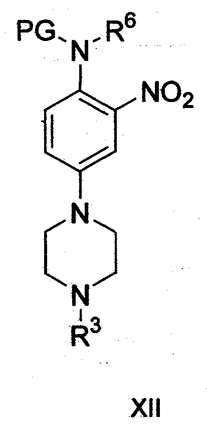 wherein R³ and R⁶ have the meaning according to one or more of claims 1 to 16 and PG represents a protecting group, preferably a-C(=O)-O-C(CH₃)₃ group, which is optionally purified and/or isolated, and the compound of general formula XII is reacted with at least one acid in a suitable reaction medium to yield a compound of general formula XIII, 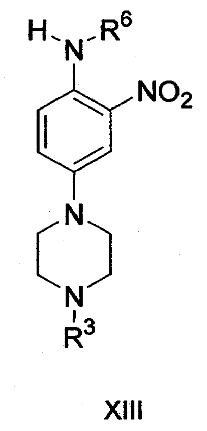 wherein R³ and R⁶ have the meaning according to one or more of claims 1 to 16, which is optionally purified and/or isolated, and the compound of general formula XIII is reacted with hydrogen in the presence of at least one base to yield a compound of general formula XIV, 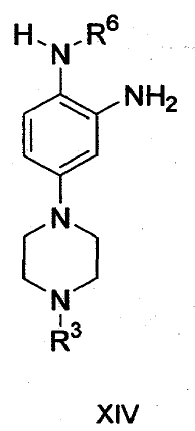 wherein R³ and R⁶ have the meaning according to one or more of claims 1 to 16, which is optionally purified and/or isolated, and the compound of general formula XIV is reacted with at least one compound of general formula R⁷⁻C(=O)-O-C(=O)-R⁷, wherein R⁷ has the meaning according to one or more of claims 1 to 16, and/or at least one compound of general formula R¹⁰-C(=O)-O-C(=O)-R¹⁰, wherein R¹⁰ has the meaning according to one or more of claims 1 to 16, in a suitable reaction medium, optionally in the presence of at least one base, to yield a compound of general formula I, wherein X represents -NR⁶⁻C(=O)R⁷, R¹ represents a hydrogen atom, R² represents a hydrogen atom or a -C(=O)-R¹⁰-moiety and R³, R⁶, R⁷ and R¹⁰ have the meaning according to one or more of claims 1 to 16, which is optionally purified and/or isolated,
or at least one compound of general formula VI, wherein X represents -CN, - C(=O)-OR⁴ or -O-R⁵, R¹ and R² have the meaning according to one or more of claims 1 to 16 or one of them represents a protecting group, preferably a - C(=O)-O-C(CH₃)₃-group, R³, R⁴ and R⁵ have the meaning according to one or more of claims 1 to 16, is reacted with at least one acid in a suitable reaction medium to yield a compound of general formula I, wherein X represents -CN, - C(=O)-OR⁴ or -O-R⁵, R¹ and R³ to R⁵ have the meaning according to one or more of claims 1 to 16 and R² represents hydrogen, which is optionally purified and/or isolated,
and optionally at least one compound of general formula I, wherein X represents -CN, -C(=O)-OR⁴ or -O-R⁵, R¹ and R³ to R⁵ have the meaning according to one or more of claims 1 to 16 and R² represents hydrogen, is reacted with hydrogen in the presence of at least one catalyst in a suitable reaction medium to yield a compound of general formula I, wherein X represents -CN, -C(=O)-OR⁴ or -O-R⁵, R³ to R⁵ have the meaning according to one or more of claims 1 to 16 and R¹ and R² each represent hydrogen,
or
at least one compound of general formula VI, wherein X represents -C(-O)-OR⁴, R¹ and R² have the meaning according to one or more of claims 1 to 16 or one of them represents a protecting group, preferably a -C(=O)-O-C(CH₃)₃₋group, R³ and R⁴ have the meaning according to one or more oficlaims 1 to 16, is reacted with at least one base in a suitable reaction medium to yield a compound of general formula XV, wherein X represents -C(=O)-OH, 'R¹ and R² have the meaning according to one or more of claims 1 to 16 or one of them represents a protecting group, preferably a -C(=O)-O-C(CH₃)₃-group, R³ has the meaning according to one or more of claims 1 to 16, which is optionally purified and/or isolated and at least one compound of general formula XV is reacted with at least one acid in a suitable reaction medium to yield a compound of general formula I, wherein X represents -C(=O)-OH, R¹ and R³ have the meaning according to one or more of claims 1 to 16 and R² represents hydrogen, which is optionally purified and/or isolated,
or
at least one compound of general formula VI, wherein X represents -NO₂, R¹ and R² have the meaning according to one or more of claims 1 to 16 or one of them represents a protecting group, preferably a -C(=O)-O-C(CH₃)₃-group, R³ has the meaning according to one or more of claims 1 to 16, is reacted with hydrogen in the presence of at least one catalyst in a suitable reaction medium to yield a compound of general formula XVI, wherein X represents -NH₂, R¹ and R² have the meaning according to one or more of claims 1 to 16 or one of them represents a protecting group, preferably a -C(=O)-O-C(CH₃)₃-group, R³ has the meaning according to one or more of claims 1 to 16, which is optionally purified and/or isolated, and at least one compound of general formula XVI, is reacted with at least one acid in a suitable reaction medium to yield a compound of general formula I, wherein X represents -NH₂, R¹ and R³ have the meaning according to one or more of claims 1 to 16 and R² represents hydrogen, which is optionally purified and/or isolated,
and optionally at least one compound of general formula I, wherein X represents -NH₂, R¹ and R³ have the meaning according to one or more of claims 1 to 16 and R² represents hydrogen, is reacted with at least one compound of general formula R⁷-C(=O)-O-C(=O)-R⁷ and/or at least one compound of general formula R¹⁰-C(=O)-O-O(=O)-R¹⁰, wherein R⁷ and R¹⁰ have the meaning according to one or more of claims 1 to 16, in a suitable reaction medium, optionally in the presence of at least one base, to yield a compound of general formula I, wherein X represents -NH-O(=O)-R⁷ and R¹ to R³ have the meaning according to one or more of claims 1 to 16, which is optionally purified and/or isolated,
or optionally at least one compound of general formula I, wherein X represents -NH₂ and R¹ and R³ have the meaning according to one or more of claims 1 to 16 and R² represents hydrogen, is reacted with at least one compound of general formula R⁸-S(=O)-W, wherein R⁸ has the meaning according to one or more of claims 1 to 16 and W represents a halogen atom, preferably a chlorine atom, in a suitable reaction medium, optionally in the presence of at least one base, to yield a compound of general formula I,
wherein X represents -NH-S(=O)₂-R⁸ and R¹, R³ and R⁸ have the meaning according to one or more of claims 1 to 16 and R² represents hydrogen, which is optionally purified and/or isolated.

**19.** Medicament comprising at least one substituted phenyl-piperazine compound according to one or more of claims 1 to 16 and optionally at least one physiologically acceptable auxiliary agent.

**20.** Medicament according to claim 19 for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder); for improvement of cognition (cognitive enhancement) or cognitive memory (cognitive memory enhancement); for the prophylaxis and/or treatment of drug addiction and/or withdrawal; for the prophylaxis and/or treatment of alcohol addiction and/or withdrawal, for the prophylaxis and/or treatment of nicotine addiction and/or withdrawal.

**21.** Use of at least one substituted phenyl-piperazine compound according to one or more of claims 1 to 16 for the manufacture of a medicament for the prophylaxis and/or treatment of a disorder or disease related to food intake, preferably for the regulation of appetite; for the maintenance, increase or reduction of body weight; or for the prophylaxis and/or treatment of obesity, bulimia, anorexia, cachexia or type II diabetes (non insulin dependent diabetes mellitus), more preferably for the prophylaxis and/or treatment of obesity.

**22.** Use of at least one substituted phenyl-piperazine compound according to one or more of claims 1 to 16 for the manufacture of a medicament for the prophylaxis and/or treatment of stroke; migraine; head trauma; epilepsy; irritable colon syndrome; irritable bowl syndrome; disorders of the central nervous system; anxiety; panic attacks; depression; bipolar disorders; obsessive compulsory disorder; cognitive disorders; cognitive dysfunction associated with psychiatric diseases; memory disorders; senile dementia; mood disorders; sleep disorders; psychosis; neurodegenerative disorders, preferably selected from the group consisting of Morbus Alzheimer, Morbus Parkinson, Morbus Huntington and Multiple Sclerosis; schizophrenia; chronic intermittent hypoxia; convulsions; or hyperactivity disorder (ADHD, attention deficit/hyperactivity disorder).

**23.** Use of at least one substituted phenyl-piperazine compound according to one or more of claims 1 to 16 for the manufacture of a medicament for the improvement of cognition (cognitive enhancement) and/or for the improvement of cognitive memory (cognitive memory enhancement).

**24.** Use of at least one substituted phenyl-piperazine compound according to one or more of claims 1 to 16 for the manufacture of a medicament for the prophylaxis and/or treatment of drug addiction and/or withdrawal, preferably for the prophylaxis and/or treatment of addiction and/or withdrawa related to one or more of drugs selected from the group consisting of benzodiazepines, natural, semisynthetic or synthetic opioids like cocaine, ethanol and/or nicotine.
